# EUROPEAN PATENT SPECIFICATION

(11) **EP 1 584 303 B1**
(45) Date of publication and mention of the grant of the patent: **12.12.2007**
(21) Application number: 05009068.7
(22) Date of filing: 14.02.2001
(51) Int. Cl.: A61F 2/00

(54) **Hydraulic anal incontinence treatment apparatus**
Hydraulisches Gerät zur Behandlung analer Inkontinenz
Appareil hydraulique destiné au traitement de l'incontinence anale

(30) Priority: 14.02.2000 US 503483
(43) Date of publication of application: 12.10.2005
(62) Divisional of application: 01904758.8
(73) Proprietor: Potencia Medical AG, 6341 Baar (CH)
(72) Inventor: Forsell, Peter, 6300 Zug (CH)
(74) Representative: Strandin, Heléne

(56) References cited:
- EP-A- 0 314 258
- US-A- 4 056 095

## Description

The present invention relates to anal incontinence treatment apparatus, comprising an adjustable restriction device adapted to engage the colon, rectum or anus of a patient, who suffers from anal incontinence, to form a restricted faecal passageway in the colon, rectum or anus. An implantable adjustment device is provided for adjusting the restriction device to restrict the colon, rectum or anus to close the faecal passageway, or release the colon, rectum or anus to open the faecal passageway, when the restriction device is implanted in the patient. An implantable operation device is provided for operating the adjustment device. The closest prior art is US-A-4056095, which defines the preamble of claim 1

Anal incontinence is a wide-spread disease. Several kinds of sphincter plastic surgery are used today to remedy anal incontinence. There is a prior manually operated sphincter system in an initial clinical trial phase where a hydraulic sphincter system connected to an elastic reservoir (balloon) placed in the scrotum is developed. A disadvantage of this system is that thick, hard fibrosis is created around the reservoir by pump movements making the system useless sooner or later. Another disadvantage is that the use of hydraulic fluid always entails a risk of fluid leaking from the implanted hydraulic system.

Furthermore, it is a rather complicated task to manually pump the reservoir when defaecation is needed. U.S. Patent No. 5593443 discloses hydraulic anal sphincter under both reflex and voluntary control. An inflatable artificial sphincter with the pump system in scrotum is disclosed in U.S. Patent No. 4222377.

A prime object of the present invention is to provide an anal incontinence treatment apparatus, which does not require manual manipulation of a combined reservoir and pump mechanism placed in the scrotum or labia majora region of the patient.

Another object of the invention is to provide an anal incontinence treatment apparatus, which does not require complicated surgery.

Yet another object of the invention is to provide an anal incontinence treatment apparatus, which may be conveniently remote controlled by the patient.

These objects are obtained by the invention as defined in claim 1..

The expression "powered" should be understood as energised with everything without manual force, preferably electric energy. In other words, the adjustment device is operated in a non-manual manner. The expression "non-manual manner" should be understood to mean that the adjustment device is not operated by manually touching subcutaneously implanted components of the apparatus or not manipulated by touching the skin of the patient. Thus, as opposed to prior practice when treating anal incontinence, the adjustment device of the invention is not operated by manual forces, such as by manually compressing a fluid containing balloon implanted in the scrotum or in the region of labia majora. Of course, manual manipulation of a subcutaneous start button or the like for activating the powered operation device is permitted within the scope of the present invention.

Alternatively, or in combination with a powered operation device, the servo means may be used, which enables manual manipulation without need for strong manipulation forces. The servo means may comprise hydraulic means, electric control means, magnetic means, or mechanical means, which may be activated by manual manipulating means. Using a servo system will save the use of force when adjusting the adjustment device, which may be of importance in many applications.

The term "servo means" encompasses the normal definition of a servo mechanism, i.e. an automatic device that controls large amounts of power by means of very small amounts of power, but may alternatively or additionally encompass the definition of a mechanism that transfers a weak force acting on a moving element having a long stroke into a strong force acting on another moving element having a short stroke. The servo means may comprise a motor, preferably an electric motor, which may be reversible.

In accordance with a main embodiment of the invention, the apparatus comprises a reservoir, preferably containing a predetermined amount of hydraulic fluid, also implantable in the patient, wherein the operation device, suitably electrically powered, operates the adjustment device by using the hydraulic fluid of the reservoir.

The adjustment device may comprise an expandable cavity in the restriction device, wherein the colon, rectum or anus is sqeezed upon expansion of the cavity and released upon contraction of the cavity. In this embodiment the operation device is adapted to distribute hydraulic fluid from the reservoir to expand the cavity, and from the cavity to the reservoir to contract the cavity.

A fluid distribution tube may readily be connected between the reservoir and the cavity in a manner so that the tube does not interfere with other implanted components of the apparatus.

Preferably, the reservoir defines a chamber for the predetermined amount of fluid and the operation device changes the volume of the chamber. The operation device suitably comprises first and second wall portions of the reservoir and is adapted to provide relative displacement between the first and second wall portions of the reservoir, in order to change the volume of the chamber.

The operation device may be adapted to provide said relative displacement in response to the pressure in the reservoir. Suitably, the operation device comprises a pressure controlled hydraulic operation device. For safety, an alarm may be provided for generating an alarm signal in response to the lapse of a predetermined time period during which the pressure controlling the hydraulic operation device exceeds a predetermined high value.

Suitably, the operation device is adapted to distribute fluid from the reservoir to the cavity of the restriction member in response to a predetermined first displacement of the first wall portion of the reservoir relative to the second wall portion of the reservoir and may distribute fluid from the cavity to the reservoir in response to a predetermined second displacement of the first wall portion relative to the second wall portion.

The first and second wall portions of the reservoir may be displaceable relative to each other by a magnetic, hydraulic, or electric power means, such as an electric motor. In this embodiment no pump is used, only the volume of the reservoir is varied. This is of great advantage compared to the solution described below when the operation device comprises a pump used to pump fluid between the reservoir and the adjustment device because there is no need for a non-return valve and it is still possible to have fluid going both to and from the reservoir. Thus, the significant risk of malfunction when using such a non-return valve implanted in the patient is eliminated.

The operation device may comprise hydraulic means and a fluid conduit extending between the hydraulic means and the adjustment device. The hydraulic means and conduit are devoid of any non-return valve. The reservoir may form part of the conduit and a fluid chamber with a variable volume. The operation device may distribute fluid from the fluid chamber to the adjustment device by reduction of the volume of the chamber and withdraw fluid from the adjustment device by expansion of the volume of the chamber. The operation device preferably comprises a motor for moving a movable wall of the reservoir for changing the volume of the chamber. Any kind of motor could be used for the different operations as well as wireless remote solutions for controlling the operations.

The restriction device preferably is operable to perform a reversible function and accordingly there is a reversing device implantable in the patient for reversing the function performed by the restriction device. Such a reversing function preferably involves enlarging and restricting the faecal passageway by the restriction device, suitably in a stepless manner. In this connection, the control device suitably controls the reversing device, which may include a switch, to reverse the function performed by the restriction device. The reversing device may comprise hydraulic means including a valve for shifting the flow direction of a fluid in the hydraulic means. Alternatively, the reversing device may comprise a mechanical reversing device, such as a switch or a gearbox.

Where the reversing device comprises a switch the control device suitably controls the operation of the switch by shifting polarity of released energy supplied to the switch. The switch may comprise an electric switch and the source of energy may supply electric energy for the operation of the switch. The switch mentioned above may comprise an electronic switch or, where applicable, a mechanical switch.

Where the operation device comprises a motor, the reversing device is adapted to reverse the motor.

In accordance with another particular embodiment of the invention, the operation device comprises a pump for pumping fluid between the reservoir and the adjustment device. A mechanical solution is proposed in which it is possible to pump fluid from the reservoir to the adjustment device and *vice versa* just by pushing an activation member in one direction. The pump preferably comprises a first activation member for activating the pump to pump fluid from the reservoir to the adjustment device, and a second activation member for activating the pump to pump fluid from the adjustment device to the reservoir. At least one of the first and second activation members may be operable by manual manipulation, preferably to permit manual pushing, pulling or rotation thereof in one direction, or by a device powered magnetically, hydraulically, or electrically (e.g. by an electric motor), or be operable by a combination of these methods. Suitably, at least one of the activation members may be adapted to operate when subjected to an external pressure exceeding a predtermined magnitude.

Another alternative is a pump pumping in only one direction and an adjustable valve to change the direction of fluid to either increase or decrease the amount of fluid in the reservoir. This valve may be manipulated either manually, mechanically, magnetically, or hydraulically.

The main embodiment of the invention described above including the reservoir may alternatively be equipped with a servo means comprising a reverse servo. The term "reverse servo" is to be understood as a mechanism that transfers a strong force acting on a moving element having a short stroke into a weak force acting on another moving element having a long stroke; i.e. the reverse function of the above-defined alternative mechanism of a normal servo mechanism. A first closed hydraulic system that controls another closed hydraulic system in which hydraulic means of the adjustment device is incorporated may be used. Minor changes in the amount of fluid in a smaller reservoir of the first system could then be transferred by the reverse servo into major changes in the amount of fluid in a larger reservoir in the second system. In consequence, the change of volume in the larger reservoir of the second system affects the hydraulic means of the adjustment device. For example, a short stroke that decreases the volume of the smaller reservoir will cause the larger reservoir to supply the adjustment device with a large amount of hydraulic fluid, which in turn results in a long mechanical adjustment stroke on the restriction device.

The great advantage of using such a reverse servo is that the larger volume system could be placed inside the abdomen or retroperitoneum where there is more space and still it would be possible to use manual manipulation means of the smaller system subcutaneously. The smaller reservoir could be controlled directly or indirectly by a fluid supply means. The fluid supply means may include another small reservoir, which may be placed subcutaneously and may be activated by manual manipulation means. Both the normal servo means and the specific reverse servo may be used in connection with all of the various components and solutions described in the present specification.

Thus, the reverse servo may be adapted to provide relative displacement between the first and second wall portions of the reservoir, suitably in response to the pressure in the reservoir, in order to change the volume of the chamber of the reservoir.

Generally, the servo means, including the reverse servo, comprises a pressure controlled servo means. The alarm mentioned above may alternatively be adapted to generate an alarm signal in response to the lapse of a predetermined time period during which the pressure controlling the servo means exceeds a predetermined high value.

The reverse servo may comprise magnetic means, electric means or manual manipulation means or a combination thereof. Preferably, however, the reverse servo comprises hydraulic means.

In accordance with a particular embodiment of the invention, the reverse servo further comprises a servo reservoir defining a chamber containing servo fluid, and the operation device comprise first and second wall portions of the servo reservoir, which are displaceable relative to each other to change the volume of the chamber of the servo reservoir. The first and second wall portions of the servo reservoir may be displaceable relative to each other by magnetic means, hydraulic means, or electric control means.

Where the reverse servo comprises hydraulic means it may further comprise a fluid supply reservoir connected to the servo reservoir in a closed system and containing a further predetermined amount of fluid. The fluid supply reservoir defines a chamber for the further predetermined amount of fluid and the operation device is adapted to change the volume of the chamber and thereby control the amount of fluid in the servo reservoir. The fluid supply reservoir comprises first and second wall portions, which are displaceable relative to each other to change the volume of the chamber of the fluid supply reservoir. Suitably, the fluid supply reservoir increases the amount of fluid in the servo reservoir in response to a predetermined first displacement of the first wall portion of the fluid supply reservoir relative to the second wall portion of the fluid supply reservoir and decreases the amount of fluid in the servo reservoir in response to a predetermined second displacement of the first wall portion of the fluid supply reservoir relative to the second wall portion of the fluid supply reservoir.

In accordance with an embodiment of the invention, the adjustment device comprises a hydraulic adjustment device, and an implantable reservoir containing a predetermined amount of hydraulic fluid and a conduit providing fluid connection between the reservoir and the hydraulic adjustment device are provided. The operation device is adapted to operate the hydraulic adjustment device by distributing hydraulic fluid through the conduit between the reservoir and the hydraulic adjustment device, wherein the conduit and hydraulic adjustment device are devoid of any non-return valve to permit free flow of hydraulic fluid in both directions in the conduit. Preferably, the reservoir forms a fluid chamber with a variable volume, and the operation device is adapted to distribute fluid from the chamber to the adjustment device by reduction of the volume of the chamber and to withdraw fluid from the adjustment device by expansion of the volume of the chamber. The operation device may comprise a motor or a pump. Alternatively, the operation device may comprise a movable wall of the reservoir for changing the volume of the chamber. For example, the operation device may be adapted to change the volume of the chamber by moving the movable wall in response to the pressure in the chamber.

In the above embodiments including a reservoir for hydraulic fluid an injection port may be provided for subcutaneous implantation in the patient to be in fluid communication with the chamber of the reservoir. The injection port may be integrated in the reservoir. Such an injection port may be provided for enabling, normally single, once-and-for-all, calibration of the amount of fluid in the hydraulic system used.

In the various embodiments hereinafter described the restriction device generally forms an at least substantially closed loop. However, the restriction device may take a variety of different shapes, such as the shape of a square, rectangle or ellipse. The substantially closed loop could for example be totally flat, i.e. thin as seen in the radial direction. The shape of restriction device may also be changed during use, by rotation or movements of the restriction device in any direction. A physical lumen, like the colon, rectum or anus, often is easier to restrict by contracting two opposite sidewalls of the lumen against each other. Thus, the restriction device may be designed to perform such a contracting effect of the opposite walls of the colon, rectum or anus. Either mechanical or hydraulic solutions may be employed to operate the restriction device. Alternatively, the restriction device may comprise an adjustable cuff, a clamp or a roller for bending or rotating the colon, rectum or anus to close its passageway. Such a cuff, clamp or roller may also be utilized for squeezing the colon, rectum or anus against human material inside the body of the patient, for example the sacral bone of the patient, or against implanted structures of the apparatus. The bending or rotating members may take any shape and be either hydraulic or non-inflatable.

Preferably the restriction device comprises an elongated restriction member and forming means for forming the restriction member into at least a substantially closed loop around the colon, rectum or anus, wherein the loop defines a restriction opening, whereby the adjustment device adjusts the restriction member in the loop to change the size of the restriction opening.

Advantageously, the forming means may form the restriction member into a loop having a predetermined size. Alternatively, the forming means may form the restriction member into a loop having a size selected from several predetermined sizes.

The adjustment device may change the size of the restriction opening such that the outer circumferential confinement surface of the restriction member either is changed or is unchanged.

The elongated restriction member may be flexible, for example take the shape of a belt or cord, and the adjustment device may pull a first portion of the flexible restriction member from a second portion of the flexible restriction member opposite the first portion in the loop to squeeze the colon, rectum or anus between the opposite lengths of the elongated flexible restriction member to restrict the faecal passageway. The restriction member may be non-inflatable, and the adjustment device may mechanically adjust the restriction member in the loop.

The adjustment device may mechanically or hydraulically adjust the restriction device. In the embodiments described the adjustment device may either mechanically or hydraulically adjust the restriction device, where applicable. It should be noted that the operation device might mechanically or hydraulically operate the adjustment device irrespectively of whether the adjustment device is adapted to adjust the restriction device mechanically or hydraulically.

In accordance with an embodiment of the invention, the restriction device comprises at least two elements on opposite or different sides of the colon, rectum or anus, and the adjustment device decreases the distance between the elements to squeeze the colon, rectum or anus between the elements, thereby restricting the faecal passageway. It is also possible to use only one element and squeeze the colon, rectum or anus against human bone or tissue. The elements above may as well as all the restriction members mentioned in this application be everything from rigid to soft.

In accordance with an alternative, the restriction device bends or rotates a portion of the colon, rectum or anus to restrict the faecal passageway in the same. For example, the restriction device may comprise at least two bending members, such as cylindrical or hour-glass shaped rollers, positioned on opposite or different sides of the colon, rectum or anus and displaced relative to each other along the colon, rectum or anus, and the adjustment device may move the bending members against the colon, rectum or anus to bend the latter to restrict the faecal passageway. The restriction device may also rotate a portion of the colon, rectum or anus. The bending or rotating members may take any shape and be either hydraulic or non-inflatable.

Alternatively, the two bending members one placed more distal than the other may be rotated in opposite directions relative to each other. With interconnecting means for example flexible bands between the bending members a restriction will occur between the bending members when they are rotated.

Preferably the adjustment device is operable to adjust the restriction device to steplessly change the restriction of the faecal passageway in the colon, rectum or anus.

All embodiments according to the invention may be controlled by a wireless remote control.

In accordance with an advantageous embodiment of the invention, there is provided a wireless remote control for non-invasively controlling the operation device. The remote control may conveniently comprise an external hand-held remote control unit, which is manually operable by the patient to control the restriction device to squeeze and release the colon, rectum or anus. With the wireless remote control the apparatus of the invention is conveniently controlled by the patient when he so desires, which is of great advantage compared to the prior art procedures. With the remote control the apparatus of the invention is conveniently controlled to adjust the implanted restriction device to release the faecal passageway when the patient wants to relieve himself or herself.

The remote control may advantageously be capable of obtaining information related to important parameters, such as the condition of the faecal passageway or the pressure against the restriction device, and of commanding the operation device to operate the adjustment device to adjust the restriction device in response to obtained information. With the remote control the apparatus of the invention is conveniently controlled to adjust the implanted restriction device to open and close the faecal passageway. The adjustment device may control the restriction device to steplessly change the restriction of the passageway.

Preferably, the wireless remote control comprises a separate signal transmitter or receiver and a signal receiver or transmitter implanted in the patient. For example, the signal transmitter and signal receiver may transmit and receive a signal in the form of digital pulses, which may comprise a magnetic or electric field. Alternatively, which is preferred, the signal transmitter and signal receiver may transmit and receive an electromagnetic wave signal, a sound wave signal or a carrier wave signal for a remote control signal. The receiver may comprise an implanted control unit for controlling the adjustment device in response to a control signal from the signal transmitter. Any known or conventional signal transmitting or signal receiving means that is suitable for use with a human or mammal patient may be provided as the signal transmitter or signal receiver.

The apparatus of the invention may further comprise an implanted energiser unit for providing energy to energy consuming implanted components of the apparatus, such as electronic circuits and/or a motor for operating the adjustment device. Where a motor is provided the control unit is adapted to power the motor with energy provided by the energiser unit in response to a control signal received from the signal transmitter. The motor may be any type of motor, such as a pneumatic, hydraulic or electric motor and the energiser unit may power the motor with pressurized gas or liquid, or electric energy, depending on the type of motor. Where the motor is an electric motor, it may power pneumatic or hydraulic equipment.

The remote control advantageously comprises wireless energy transfer device for transferring energy from outside the patient's body to energy consuming implantable components of the apparatus. The energy transfer device may comprise said energiser unit is adapted to transform energy from the control signal, as it is transmitted to the signal receiver, into electric energy. Where the operation device comprises a motor the wireless energy transfer device is adapted to directly power the motor with transferred energy.

The energy transferred by the wireless energy transfer device preferably comprises a signal, suitably a wave signal. The energy transferred by the wireless energy transfer device may comprise an electric field or a magnetic field or a combination thereof. The signal may be analog or digital or a combination thereof. The energy transfer device may transfer the energy from the signal into a direct, pulsating direct or alternating current or a combination thereof.

Any of the above mentioned signals may comprise analog or digital pulses. The analog or digital signal may comprise a magnetic field or an electric field or a combination thereof. Where the signal is a wave signal it may comprise an electromagnetic wave signal, a sound wave signal or a carrier wave signal for a remote control signal or a combination thereof. Where a carrier signal is used it may be frequency, amplitude or frequency and amplitude modulated.

The apparatus of the invention may comprise an implantable source of energy for powering the operation device and/or for energizing other energy consuming components of the apparatus, wherein the energy from the source of energy is releasable from outside the patient's body. Furthermore, the apparatus may comprise an energy transmission device for wireless transmission of energy of a first form and an energy transforming device implantable in the patient for transforming the energy of the first form into energy of a second form, to be supplied to the source of energy and/or other implantable energy consuming parts of the apparatus. The energy transforming device may transform the wireless energy directly or indirectly into energy different than the wireless energy for operation of the restriction device. Typically, the energy of the second form is different than the energy of the first form. The function of the energy transmission device may be different from that of the energy transforming device.

An implantable motor or pump for operating the adjustment device may be provided, wherein the energy transmission device may be adapted to transmit wireless energy in the form of a magnetic field or electromagnetic waves or field for direct power of the motor or pump, as the wireless energy is being transmitted. Suitably, the energy transmission device transmits energy by at least one signal separate from the above mentioned control signal.

An implantable stabiliser for stabilising the energy of the first or second form may be provided. Where the energy of the second form comprises electric current, the stabiliser suitably comprises at least one capacitor.

Generally, the source of energy comprises a battery, accumulator, capacitor or a combination thereof.

In accordance with an embodiment of the invention, the apparatus comprises a control device adapted to produce wireless energy for directly powering the operation device and/or for energizing other energy consuming components of the apparatus.

It should be understood that the energy consuming parts of the apparatus for example a motor or pump may be or may not be energised with the unchanged wirelessly transmitted energy as this being transmitted as well as being or not being energised with energy different than the transmitted energy for example transformed into electrical energy but still directly used for energising the energy consuming parts of the apparatus as the transmitted energy is transmitted. Alternatively the energy consuming parts of the apparatus may be energised from a implanted source of energy or storage device, which still may be loaded with wireless energy. In all these aspects it is preferable to be able to wirelessly control the release of energy and get an feedback of the result of the performed function of the device. Direct use of transmitted energy may be unrelaible without a feedback what has happened, has the energy reached it's goal?

Generally, the wireless energy may comprise a wave signal including a sound wave signal, an ultrasound wave signal, an electromagnetic wave signal, an infrared light signal, a visible light signal, an ultra violet light signal, a laser light signal, a micro wave signal, a radio wave signal, an x-ray radiation signal or a gamma radiation signal.

Any of the above mentioned signals may comprise a wave signal including a sound wave signal, an ultrasound wave signal, an electromagnetic wave signal, an infrared light signal, a visible light signal, an ultra violet light signal, a laser light signal, a micro wave signal, a radio wave signal, an x-ray radiation signal or a gamma radiation signal.

The control device may be adapted to produce wireless energy in the form of a train of energy pulses and the energy transfer device may be adapted to intermittently transfer the train of energy pulses for direct use in connection with the energising of the energy consuming components of the apparatus. Alternatively, the control device may be adapted to control the energy transforming device to produce the energy of the second form in said train of energy pulses for direct use in connection with the operation of the adjustment device. The transferred energy preferably comprises electric energy. An implantable capacitor may be provided for producing the train of energy pulses.

Where a capacitor is used in any of the above described embodiments it may have a relatively low capacity, i.e. less than 0,1 µF, in order to be small and suited for implantation.

Where the operation device comprises an implantable motor or pump for operating the adjustment device, the energy transfer device may be adapted to directly power the motor or pump with transferred energy, at the same time as the energy is transferred. Where a pump is used it should not be a plunger type of pump, because a plunger pump is noisy, but may comprise a peristaltic or membrane pump.

As mentioned above the apparatus comprises a wireless remote control for non-invasively controlling the operation device, which preferably is electrically powered. Alternatively, the operation device is powered by magnetic energy, non-magnetic energy, electromagnetic energy, non-electromagnetic energy, kinetic energy, non-kinetic energy, sonic energy, non-sonic energy, thermal energy or non-thermal energy. However, the operation device may be unpowerable by permanent static magnetic energy. Any other kind of energy, such as electric, electromagnetic energy or a moving permanent magnetic energy, may be conceivable for operating the adjustment device. As a result, the implanted restriction device would not be accidentally adjusted if the patient comes close to any permanent magnet. Suitably, the operation device is adapted to non-invasively operate the adjustment device.

Where the operation device comprises a hydraulic operation device it may use hydraulic fluid, the viscosity of which changes when the hydraulic fluid is exposed to energy, preferably electric energy, different than thermal energy. However, use of hydraulic fluid of the kind having a viscosity that substantially increases when exposed to heat or a magnetic field, i.e. the hydraulic fluid would not become more viscous when exposed to heat or influenced by magnetic forces, should be avoided, because external heat sources or heat from the body when the patient has fever and external magnetic sources might affect the implanted components of the apparatus.

The adjustment device is may be operable to adjust the restriction device to steplessly change the restriction of the faecal passageway. Furthermore, the adjustment device may be adapted to mechanically adjust the restriction device. Alternatively, it may be adapted to hydraulically adjust the restriction device by using hydraulic means, which is devoid of hydraulic fluid of the kind having a viscosity that substantially increases when exposed to heat or a magnetic field.

In accordance with an embodiment of the invention, the apparatus comprises a control device for controlling the restriction device. The control device may comprise an internal programmable control unit implantable in the patient and, possibly an external control unit outside the patient's body for programming the programmable internal control unit. Alternatively, the external control unit may be programmable and wirelessly control the restriction device. The control device may be adapted to produce wireless energy for directly powering the operation device and/or for energizing other energy consuming components of the apparatus.

At least one sensor for sensing at least one physical parameter of the patient may conveniently be implanted in the patient. The sensor may preferably sense as the physical parameter the horizontal position of the patient or may comprise a pressure sensor for sensing the pressure against the restriction device or the colon, rectum or anus or other important parameters. The pressure sensor may be any suitable known or conventional pressure sensor such as shown in U.S. patents 5540731, 4846181, 4738267, 4571749, 4407296 or 3939823; or an NPC-102 Medical Angioplasty Sensor.

Either the internal control unit or the external control unit of the control device may suitably control the restriction device to enlarge or close the faecal passageway. For safety the restriction device may enlarge or open the faecal passageway in response to the sensor sensing for example an abnormally high pressure value. The internal control unit may directly control the restriction device in response to signals from the sensor.

Wherever magnetic means is utilized according to the invention it may comprise a permanent magnet and a magnetic material reed switch, or other suitable known or conventional magnetic means.

Where a source of energy is used the control device suitably is operable from outside the patient's body for controlling the source of energy to release energy for use in connection with the operation of the adjustment device, when the adjustment device is implanted. The source of energy may be provided external to the patient's body, and the control device may be adapted to control the external source of energy to release wireless energy for use in connection with the operation of the adjustment device.

The control device may control the source of energy to release magnetic energy, non-magnetic energy, electromagnetic energy, non-electromagnetic energy, kinetic energy, non-kinetic energy, sonic energy, non-sonic energy, thermal energy or non-thermal energy, preferably in a non-invasive manner and for a determined time period and/or in a determined number of energy pulses.

Where the implantable components of the apparatus comprise electrical electrical components they may include at least one or a single voltage level guard. In this case, the electrical components suitably are devoid of any current detector and/or charge level detector. Furthermore, the electrical components may comprise a capacitor or accumulator, wherein the charge and discharge of the capacitor or accumulator is controlled by use of the voltage level guard. As a result, there is no need for any implanted current detector and/or charge level detector for the control of the capacitor, which makes the apparatus simple and reliable.

In accordance with an advantageous embodiment of the invention, the apparatus comprises an implantable switch for directly or indirectly switching the operation of the restriction device. The switch may be operated by the energy supplied by the energy transmission device mentioned above to switch from an off mode, in which the implantable source of energy mentioned above is not in use, to an on mode, in which the source of energy supplies energy for the operation of the restriction device.

In accordance with an alternative embodiment, the above mentioned a remote control may be employed for controlling the implantable source of energy, wherein the switch is operated by the energy supplied by the energy transmission device to switch from an off mode, in which the remote control is prevented from controlling the source of energy and the source of energy is not in use, to a standby mode, in which the remote control is permitted to control the source of energy to supply energy for the operation of the restriction device.

In accordance with another alternative embodiment, the switch is operated by the energy supplied by the implantable energy transforming device mentioned above to switch from an off mode, in which the source of energy is not in use, to an on mode, in which the source of energy supplies energy for the operation of the restriction device.

In accordance with yet another alternative embodiment, the switch is operated by the energy supplied by the energy transforming device to switch from an off mode, in which the remote control is prevented from controlling the source of energy and the source of energy is not in use, to a standby mode, in which the remote control is permitted to control the source of energy to supply energy for the operation of the restriction device.

Suitably, the restriction device is embedded in a soft or gel-like material, such as a silicone material having hardness less than 20 Shore.

The energy transforming device may be designed to be implanted subcutaneously or in the abdomen, thorax or cephalic region of the patient.

The adjustment device may be adapted to adjust the restriction device such that the restriction device provides a predetermined contraction of the faecal passageway that is satisfactory for the patient.

The adjustment device is preferable adapted to adjust the prosthesis device in a non-flux magnetic or non-thermal manner or non-viscosity changing manner because these gives an unrelaible function of the device. With non-viscosity changing manner should be understood when the adjustment device primary is adjusted with the change of the viscosity of hydraulic fluid.

All the above described various components, such as the motor, pump and capacitor, may be combined in the different embodiments where applicable. Also the various functions described in connection with the above embodiments of the invention may be used in different applications, where applicable. Specifically, the various remote control functions described and all the various methods for supplying energy may be used in any conceivable combination that is apparent to those skilled in the art.

A patient suffering from anal incontinence comprises the steps of placing at least two laparascopical trocars in the body of a patient suffering from anal incontinence, inserting a dissecting tool through the trocars, and dissecting an area of the colon, rectum or anus in the abdominal or pelvic or retroperitoneal surroundings, placing at least one adjustable restriction device in the dissected area engaging the rectum or colon or anus, adjusting the restriction device to normally restrict the faecal passageway in the rectum or colon or anus, and adjusting the restriction device to open the faecal passageway when the patient wants to relieve himself or herself. A hydraulic adjustable restriction device may be used when practising this method, in a non-manual manner, i.e. without touching subcutaneously implanted components of the apparatus or by using power means that is manually activated.

The method may further comprise implanting a source of energy in the patient and providing a control device for controlling the source of energy from outside the patient's body to supply energy for the adjustment of the restriction device.

A method for treating anal incontinence comprises surgically implanting in the body of a patient suffering from anal incontinence an adjustable restriction device engaging the colon, rectum or anus to restrict the faecal passageway, and when desired to allow the patient to relieve himself or herself, adjusting the restriction device to temporarily release the colon, rectum or anus to open the faecal passageway. The method may further comprise implanting an elongated restriction member of the restriction device around the colon, rectum or anus.

The anal incontinence treatment apparatus of the invention may also be laparoscopicly implanted. Thus, there is provided a method comprising the steps of placing at least two laparascopical trocars in the patient's body, inserting a dissecting tool through the trocars and dissecting an area of the pelvic or abdominal or retroperitoneal surroundings and placing an operable restriction device in the dissected area, so that the restriction device engages the colon, rectum or anus to restrict the faecal passageway. Additionally, the method may comprise implanting an energy transfer device of the apparatus.

The invention is described in more detail in the following with reference to the accompanying drawings, in which
FIGURES 1A-D are block diagrams of four different principal embodiments of the anal incontinence treatment apparatus according to the invention.
FIGURE. 2A-D are cross-sectional views of a pump mechanism according to Fig. 1C, which is designed to pump fluid in opposite directions by mechanically pushing a wall portion in only one direction.
FIGURE 3 is a cross-sectional view of a reservoir having a variable volume controlled by a remote control motor, in accordance with a particular embodiment of the principal embodiment shown in Fig. 1B or 2B.
FIGURE 4 is a cross-sectional view of a reservoir having a variable volume adjustable by manual manipulation, in accordance with a particular embodiment of the principal embodiment shown in Fig. 1B or 1D.
FIGURE 5A is a perspective view of a hydraulic, pneumatic or mechanical servo system in accordance with a particular embodiment of the principal embodiment shown in Fig. 1D.
FIGURE 5B is a cross-sectional view taken along line VB-VB of Fig 5A.
FIGURE 6 is a block diagram illustrating remote control components of the device of the invention;
FIGURE 7 is a schematic view of exemplary circuitry used for the block diagram in Fig. 4;
FIGURE 8 is a schematic view af a band with a cavity defining a restriction opening for use in accordance with the invention.
FIGURES 9A and 9B are schematic views of a first mechanical restriction device for use in accordance with the invention;
FIGURES 10A and 10B are schematic views of a second mechanical restriction device for use in accordance with the invention;
FIGURE 11 is a schematic view of a third mechanical restriction device for use in accordance with the invention;
FIGURE 12A is a schematic front view of a fourth mechanical restriction device for use in accordance with the invention;
FIGURES 12B and 12C are sectional views along the line A-A of FIGURE 12A;
FIGURES 13A through 17B are five modifications of the embodiment of FIGURES 12A-12C;
FIGURE 18 is a view of an inflatable restriction device of the apparatus of the invention: and
FIGURE 19 illustrates the apparatus of the invention implanted in a patient.

Referring to the drawing figures, like reference numerals designate identical or corresponding elements throughout the several figures.

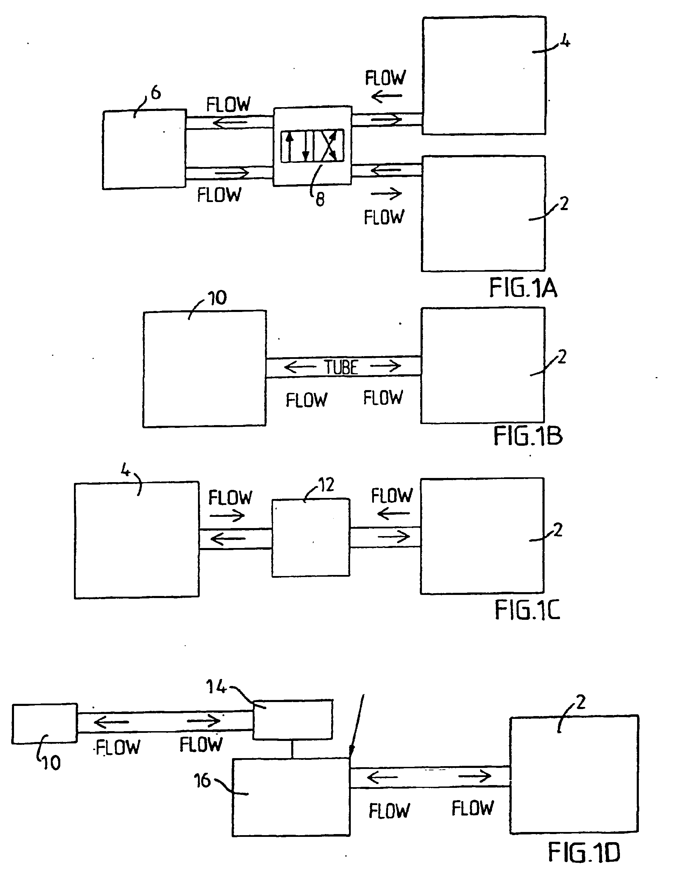

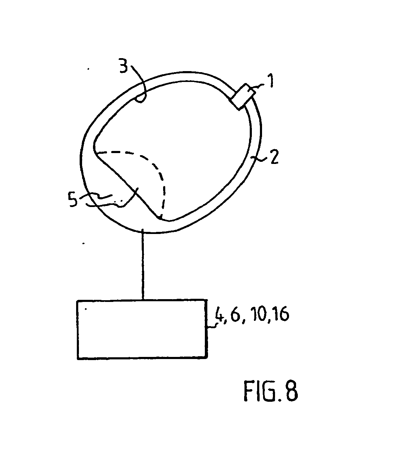

FIGURES 1A-D is a block diagram of four different embodiments of the anal incontinence treatment apparatus according to the invention. FIGURE 1A shows an elongated restriction member in the form of a band 2 forming a loop which defines a restriction opening. The band 2 provides a restricted faecal passageway in the rectum when applied around the latter. FIGURE 1A further shows a separate reservoir 4, a one way pump 6 and an alternate valve 8. FIGURE 1B shows the band 2 and a fluid supply reservoir 10. FIGURE 1C shows the band 2, a two way pump 12 and the reservoir 4. FIGURE 1D shows a servo system with a first closed system controlling a second system. The servo system comprises the fluid supply reservoir 10 and a servo reservoir 14. The servo reservoir 14 controls a larger adjustable reservoir 16 which in connection with the band 2 applied around the rectum varies the volume of a cavity in the band, which in turn varies the restricted faecal passageway in the rectum. Such a band 2 forming the restriction opening 3 is illustrated schematically in FIGURE 8. The band 2 comprises an adjustment device having an expandable/contractabe cavity 5 which is expanded or contracted by supplying hydraulic fluid (e.g. from reservoir 4, 6, 10, or 16), and the band 2 may be sutured in place, illustrated schematically at 7 in FIGURE 8.

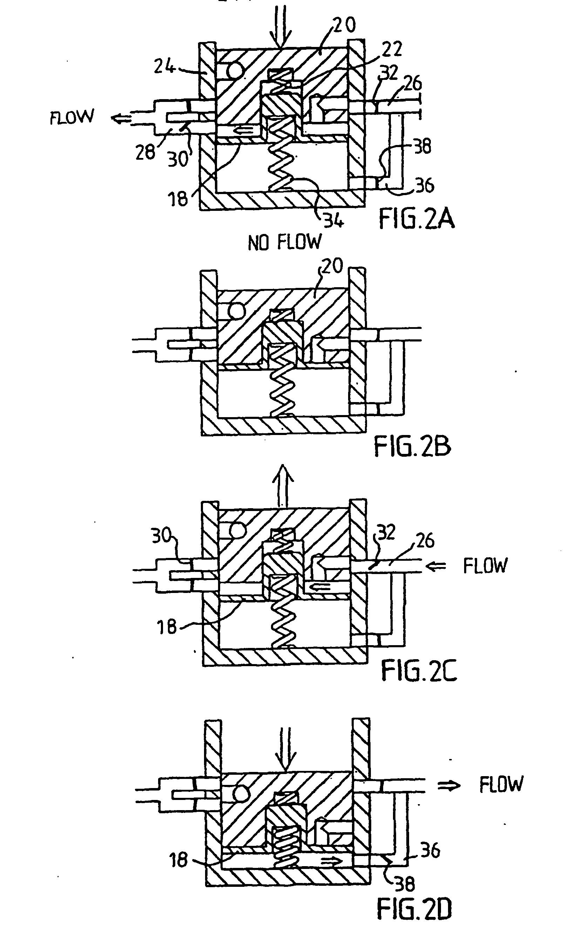

FIGURES 2A-D are cross-sectional views of a pump mechanism adapted to pump fluid in both directions only by mechanically pushing a separate sealing wall portion 18 in one direction. FIGURE 2A shows a piston 20 pushed forwards against a spring 22 towards the wall portion 18 and located in a pump housing 24 conducting fluid from a right upper fluid passage 26 of the housing 24 to a left fluid passage 28 of the housing 24. A main valve 30 is open and a nonreturn valve 32 is closed. FIGURE 2B illustrates the first pump movement in which the piston 20 has moved forwards and reaches the wall portion 18. FIGURE 2C illustrates how the piston 20 moves backwards by the action of the spring 22. The main valve 30 is now closed and the nonreturn valve 32 is open for fluid from the right upper passage 26. FIGURE 1D illustrates how the piston 20 is moved further downwards from its position according to FIGURE 2B while pushing the wall portion 18 downwardly against a second spring 34 that is stronger than spring 22, whereby fluid escapes from a right lower fluid passage 36. When moving the piston 20 backwardly from the position according to FIGURE 2D, fluid enters the left fluid passage 28 and a valve 38 in the lower right fluid passage 36 closes.

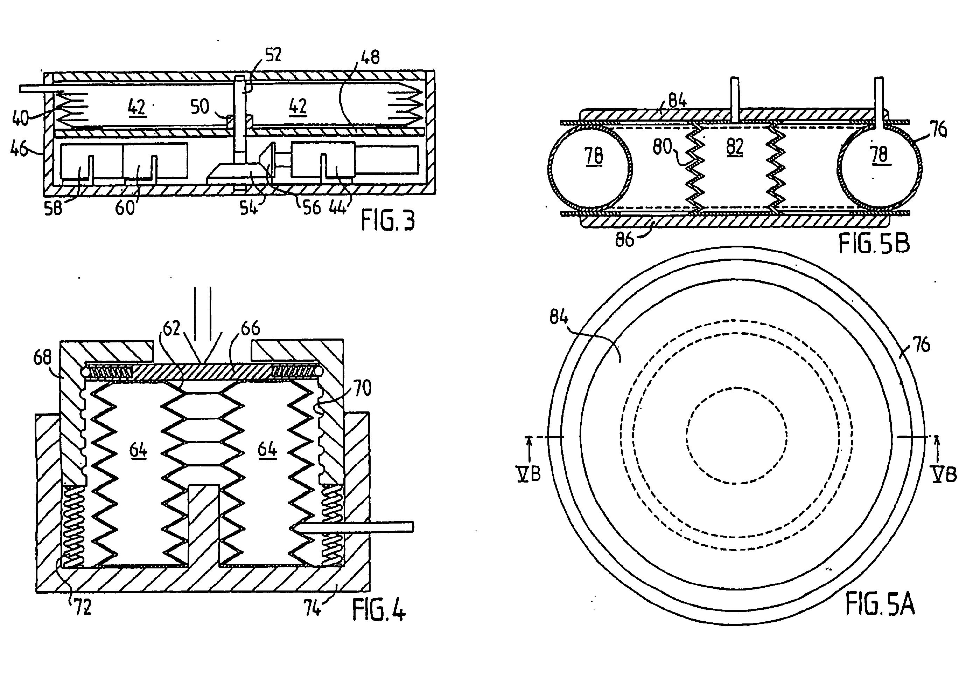

FIGURE 3 is a cross-sectional view of a reservoir 40 defining a chamber 42, the size of which is variable by an operation device comprising a remote controlled electric motor 44, in accordance with FIGURE 1B or 1D. The reservoir 40 and the motor 44 are placed in a housing 46. The chamber 42 is varied by moving a large wall 48. The wall 48 is secured to a nut 50, which is threaded on a rotatable spindle 52. The spindle 52 is rotated by the motor 44 via an angular gearing, which comprises two conical gear wheels 54 and 56 in mesh with each other. The motor 44 is powered by a battery 58 placed in the housing 46. An signal receiver 60 for controlling the motor 44 is also placed in the housing 46. Alternatively, the battery 58 and the signal receiver 60 may be mounted in a separate place. The motor 44 may also be powered by energy transferred from transmitted signals.

FIGURE 4 is a cross-sectional view of a reservoir 62 defining a chamber 64, the size of which is variable and is controlled by manual manipulation. A gable wall portion 66 of an open ended inner cylindrical housing 68 is adapted to be pushed downwards to fit in a desired locking groove 70 of a plurality of locking grooves 70 on the mantle wall of the cylindrical housing 68, to reduce the volume of the chamber 64. The inner cylindrical housing 68 is suspended by springs 72 and is telescopically applied on an outer cylindrical housing 74. When pushing the inner cylindrical housing 68 it moves downwards relative to the outer cylindrical housing 74 causing the gable wall portion 66 to release from the locking groove 70 and move upwards relative to the inner cylindrical housing 68. When the inner housing 68 is moved upwardly by the action of the springs 72 the volume of the chamber 64 is increased.

FIGURES 5A and 5B show a servo means comprising a main ring-shaped fluid reservoir 76 defining a chamber 78, the size of which is variable. Centrally positioned in the main ring-shaped reservoir 76 there is a servo fluid reservoir 80 defining a chamber 82, the size of which is variable. The chamber 82 of the servo reservoir 80 is substantially smaller than the chamber 78 of the main reservoir 76. The two reservoirs 76 and 80 are situated between two opposite separate walls 84 and 86, and are secured thereto. When changing the amount of fluid in the servo reservoir 80, the two opposite walls 84,86 are moved towards or away from each other, whereby the volume of the chamber 78 of the main reservoir 76 is changed.

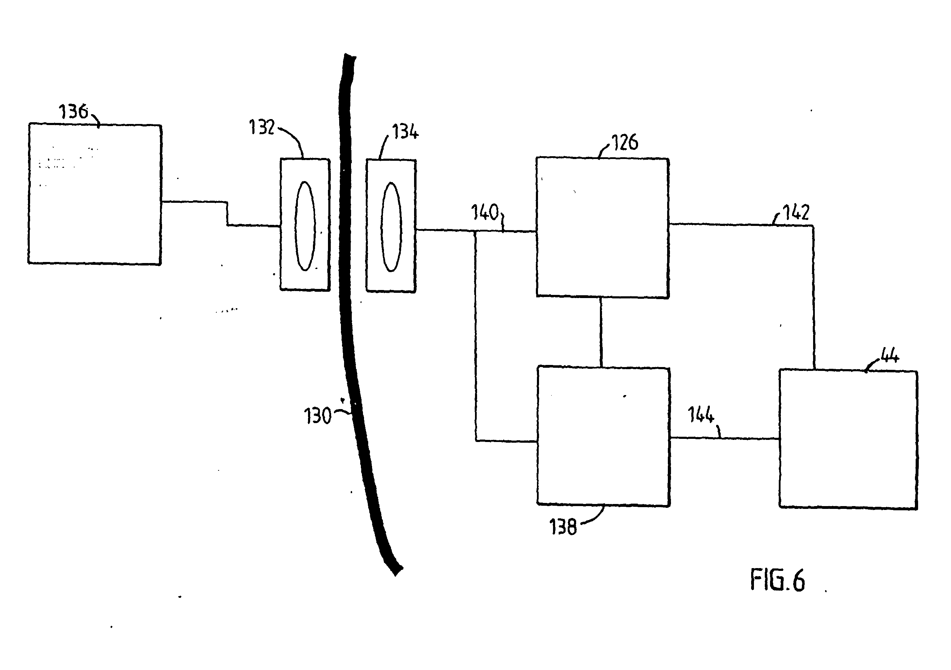

FIGURE 6 shows the basic parts of a remote control system of the apparatus of the invention including the electric motor 44 of the embodiment shown in FIGURE 3. In this case, the remote control system is based on the transmission of electromagnetic wave signals, often of high frequencies in the order of 100 kHz - 1 gHz, through the skin 130 of the patient. In FIGURE 6, all parts placed to the left of the skin 130 are located outside the patient's body and all parts placed to the right of the skin 130 are implanted. Any suitable remote control system may be used.

An external signal transmitting antenna 132 is to be positioned close to a signal receiving antenna 134 implanted close to the skin 130. As an alternative, the receiving antenna 134 may be placed for example inside the abdomen of the patient. The receiving antenna 134 comprises a coil, approximately 1-100 mm, preferably 25 mm in diameter, wound with a very thin wire and tuned with a capacitor to a specific high frequency. A small coil is chosen if it is to be implanted under the skin of the patient and a large coil is chosen if it is to be implanted in the abdomen of the patient. The transmitting antenna 132 comprises a coil having about the same size as the coil of the receiving antenna 134 but wound with a thick wire that can handle the larger currents that is necessary. The coil of the transmitting antenna 132 is tuned to the same specific high frequency as the coil of the receiving antenna 134.

An external control unit 136 comprises a microprocessor, a high frequency electromagnetic wave signal generator and a power amplifier. The microprocessor of the control unit 136 is adapted to switch the generator on/off and to modulate signals generated by the generator to send digital information via the power amplifier and the antennas 132,134 to an implanted control unit 138. To avoid that accidental random high frequency fields trigger control commands, digital signal codes are used. A conventional keypad placed on the external control unit 136 is connected to the microprocessor thereof. The keypad is used to order the microprocessor to send digital signals to either increase or decrease the size of the restriction opening defined by the loop of the restriction member 2. The microprocessor starts a command by applying a high frequency signal on the antenna 132. After a short time, when the signal has energized the implanted parts of the control system, commands are sent to increase or decrease the size of the restriction opening of the restriction member 2 in predefined steps. The commands are sent as digital packets in the form illustrated below.

| | | | |
|---|---|---|---|
| Start pattern, 8 bits | Command, 8 bits | Count, 8 bits | Checksum, 8 bits |

The commands are sent continuously during a rather long time period (e.g. about 30 seconds or more). When a new increase or decrease step is desired the Count byte is increased by one to allow the implanted control unit 138 to decode and understand that another step is demanded by the external control unit 136. If any part of the digital packet is erroneous, its content is simply ignored.

Through a line 140, an implanted energiser unit 126 draws energy from the high frequency electromagnetic wave signal received by the receiving antenna 134. The energiser unit 126 stores the energy in a power supply, such as a large capacitor, powers the control unit 138 and powers the electric motor 44 via a line 142.

The control unit 138 comprises a demodulator and a microprocessor. The demodulator demodulates digital signals sent from the external control unit 136. The microprocessor of the control unit 138 receives the digital packet, decodes it and, provided that the power supply of the energiser unit 126 has sufficient energy stored, sends a signal via a signal line 144 to the motor 44 to either increase or decrease the size of the restriction opening of the restriction member 2 depending on the received command code.

Alternatively, the energy stored in the power supply of the energiser unit may only be used for powering a switch, and the energy for powering the motor 44 may be obtained from another implanted power source of relatively high capacity, for example a battery. In this case the switch is adapted to connect the battery to the control unit 138 in an on mode when the switch is powered by the power supply and to keep the battery disconnected from the control unit in a standby mode when the switch is unpowered.

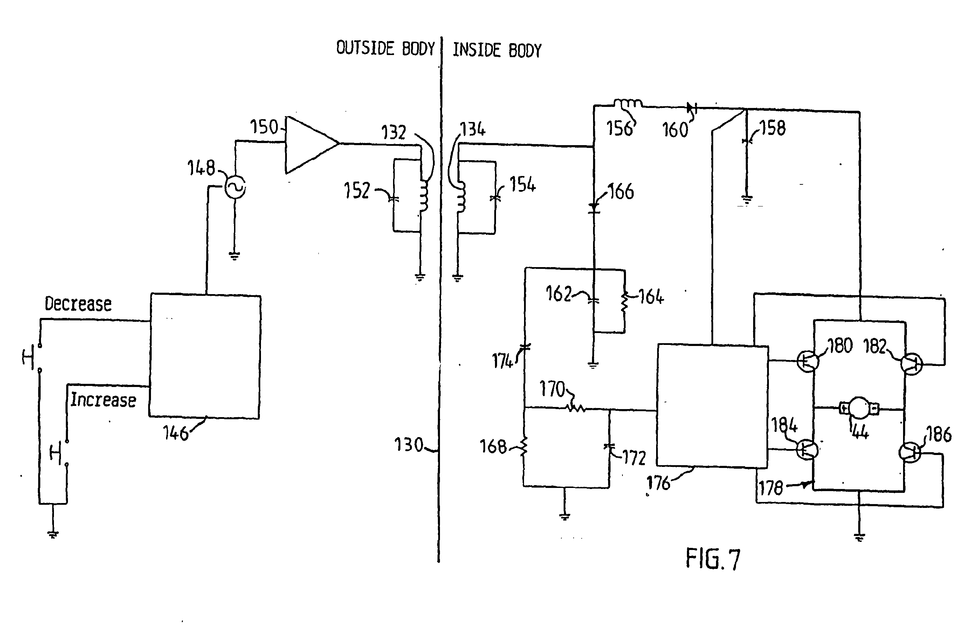

With reference to FIGURE 7, the remote control system schematically described above will now be described in accordance with a more detailed embodiment. The external control unit 136 comprises a microprocessor 146, a signal generator 148 and a power amplifier 150 connected thereto. The microprocessor 146 is adapted to switch the signal generator 148 on/off and to modulate signals generated by the signal generator 148 with digital commands that are sent to implanted components of the anal incontinence apparatus. The power amplifier 150 amplifies the signals and sends them to the external signal transmitting antenna 132. The antenna 132 is connected in parallel with a capacitor 152 to form a resonant circuit tuned to the frequency generated by the signal generator 148.

The implanted signal receiving antenna coil 134 forms together with a capacitor 154 a resonant circuit that is tuned to the same frequency as the transmitting antenna 132. The signal receiving antenna coil 134 induces a current from the received high frequency electromagnetic waves and a rectifying diode 160 rectifies the induced current, which charges a storage capacitor 158. A coil 156 connected between the antenna coil 134 and the diode 160 prevents the capacitor 158 and the diode 160 from loading the circuit of the signal receiving antenna 134 at higher frequencies. Thus, the coil 156 makes it possible to charge the capacitor 158 and to transmit digital information using amplitude modulation.

A capacitor 162 and a resistor 164 connected in parallel and a diode 166 forms a detector used to detect amplitude modulated digital information. A filter circuit is formed by a resistor 168 connected in series with a resistor 170 connected in series with a capacitor 172 connected in series with the resistor 168 via ground, and a capacitor 174, one terminal of which is connected between the resistors 168,170 and the other terminal of which is connected between the diode 166 and the circuit formed by the capacitor 162 and resistor 164. The filter circuit is used to filter out undesired low and high frequencies. The detected and filtered signals are fed to an implanted microprocessor 176 that decodes the digital information and controls the motor 44 via an H-bridge 178 comprising transistors 180,182,184 and 186. The motor 44 can be driven in two opposite directions by the H-bridge 178.

The microprocessor 176 also monitors the amount of stored energy in the storage capacitor 158. Before sending signals to activate the motor 44, the microprocessor 176 checks whether the energy stored in the storage capacitor 158 is enough. If the stored energy is not enough to perform the requested operation, the microprocessor 176 waits for the received signals to charge the storage capacitor 158 before activating the motor 44.

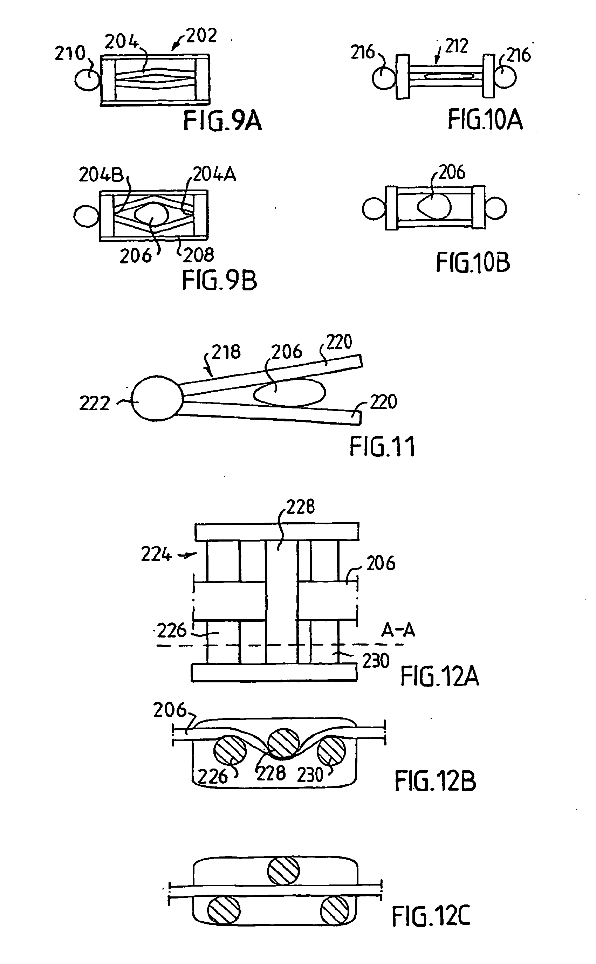

FIGURES 9A and 9B show an embodiment of the apparatus of the invention comprising a restriction device 202 having an elongated flexible restriction member 204, such as a belt, a cord or the like. The flexible member 204 extends in a loop around the rectum. (Alternatively, the flexible member 204 may comprise two separate parts on opposite sides of the rectum.) One portion 204A of member 204 is attached to a frame 208 and another portion 204B of member 204 opposite portion 204A in the loop of the flexible member 204 is connected to an adjustment device 210, which is fixed to the frame 208. The adjustment device 210 pulls the flexible member 204 away from portion 204A to squeeze the rectum between two opposite lengths of the flexible member 204 to thereby restrict the faecal passageway, see FIGURE 96A, and releases the rectum from the flexible member 204 to thereby increase the faecal passageway, see FIGURE 9B.

FIGURES 10A and 10B show an embodiment of the apparatus of the invention comprising a restriction device 212 having two plate or bar elements 214 on opposite sides of the rectum 206. An adjustment device 216 moves the elements 212 in parallel towards each other to squeeze the rectum 206 between the elements 212 to thereby restrict the faecal passageway, see FIGURE 10A, and moves the elements 212 away from each other to increase the faecal passageway, see FIGURE 10B.

FIGURE 11 shows an embodiment of the apparatus of the invention comprising a restriction device 218 having two articulated clamping elements 220 positioned on opposite sides of the rectum 206. An adjustment device 222 moves the clamping elements 220 toward each other to clamp the rectum 206 between the clamping elements 220 to thereby restrict the faecal passageway, and moves the clamping elements 220 away from each other to release the rectum 206 from the clamping elements 220 to thereby increase the faecal passageway.

FIGURES 12A, 12B and 12C show an embodiment of the apparatus of the invention comprising a restriction device 224 having three bending members in the form of cylindrical rollers 226, 228 and 230 displaced relative one another in a row along the rectum 206 and positioned alternately on opposite sides of the rectum 206. (Alternatively, each roller 226, 228 and 230 may take the shape of an hour-glass.) An adjustment device 232 moves the two outer rollers 226,230 laterally against the rectum 206 in one direction and the intermediate roller 228 against the rectum 206 in the opposite direction to bend the rectum to thereby restrict the faecal passageway, see FIGURE 12B. To release the rectum from the rollers 226-230, the adjustment device 232 moves the rollers 226-230 away from the rectum 206, see FIGURE 12C.

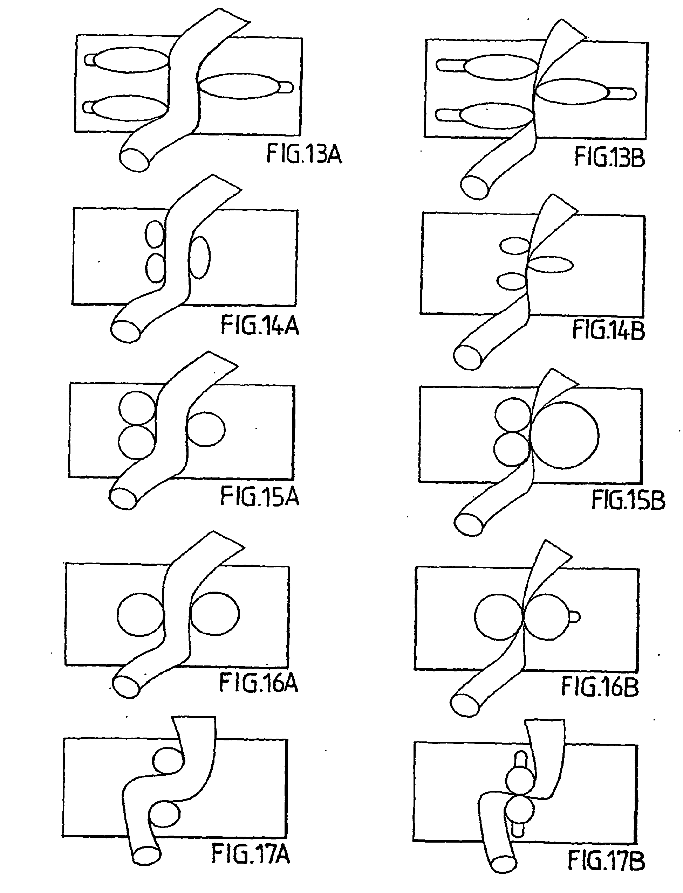

FIGURES 13A through 17B schematically illustrates modifications of the above embodiment according to FIGURES 12A-12C. Thus, FIGURES 13A and 13B show an embodiment similar to that of FIGURES 12A-12C except that the bending members are oval and not rotatable.

FIGURES 14A and 14B show an embodiment similar to that of FIGURES 13A and 13B except that the oval bending members are rotatable to squeeze the rectum, see FIGURE 14B, and to release the rectum, see FIGURE 14A.

FIGURES 15A and 15B show an embodiment similar to that of FIGURES 12A-12C except that the intermediate roller has a changeable diameter to squeeze the rectum, see FIGURE 15B, and to release the rectum, see FIGURE 15A.

FIGURES 16A and 16B show an embodiment similar to that of FIGURES 10A-10C except that the elements are replaced by two cylindrical rollers positioned on opposite sides of the rectum.

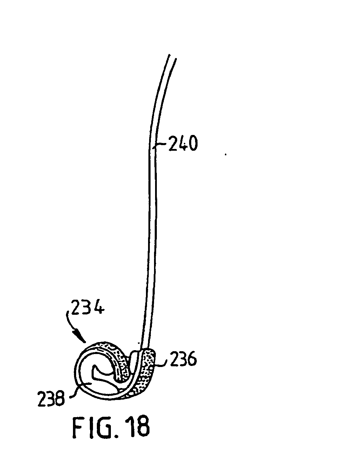

Finally, FIGURES 17A and 17B show an embodiment substantially similar to that of FIGURES 16A and 16B except that the restriction device is turned 90° to form a S-shaped curvature of the rectum.

FIGURE 18 shows an example of a hudraulic restriction device 234 for use in accordance with the invention. The restriction device 234 comprises an elongated restriction member 236 having an inflatable cavity 238. A tube 240 connects the cavity 238 to a hydraulic fluid reservoir, not shown. The restriction member 236 may be wrapped around the rectum.

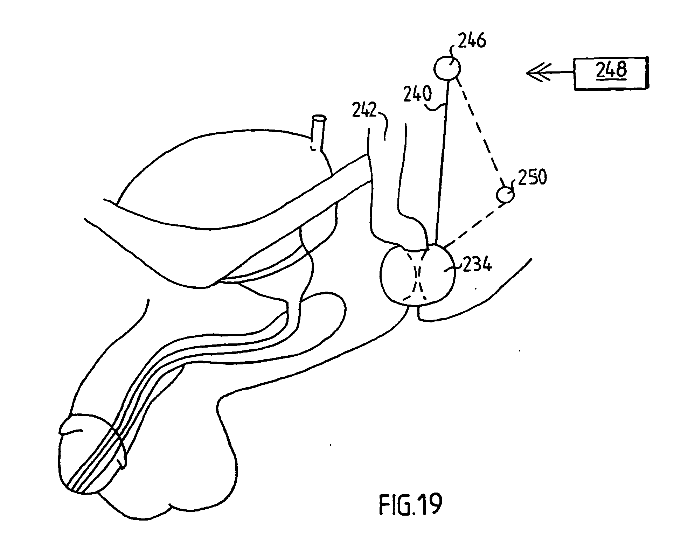

FIGURE 19 schematically illustrates how any of the above-described embodiments of the anal incontinence treatment apparatus of the invention can be implanted in a patient. Thus, an implanted adjustable hydraulic restriction device 234 extends around the rectum 242 to be capable of squeezing the rectum 242. An adjustment device in the form of an inflatable cavity in the restriction device 234 is adapted to adjust the restriction device 234 so that the faecal passageway is restricted. An implanted assembly 246 includes a hydraulic fluid reservoir and an operation device (which may include a pump) for distributing hydraulic fluid between the reservoir and the inflatable/contractible cavity of the restriction device 234 via a fluid conduit 240. A wireless remote control of the apparatus comprises an external signal transmitter 248, which may comprise a hand-held unit, and an implanted signal receiver, which is incorporated in the implanted assembly 246, includes a control unit for controlling the restriction device 234 in response to a control signal from the external transmitter. The signal receiver of the assembly 246 further includes an energiser unit which transforms energy from the control signal transmitted by the external transmitter into electric energy for energy consuming implanted components of the apparatus.

A pressure sensor 250 is implanted for sensing the pressure on the restriction device 234. The control unit of the signal receiver of the implanted assembly 246 controls the restriction device 234 to release the restriction device 234 in response to the pressure sensor 250 sensing an abnormal high pressure.

There are a number of conceivable alternative embodiments of the invention that give the same result as the above-described embodiments. For example, the microprocessor of the external and implanted, respectively, control units may be replaced by discrete components. The power amplifier of the external control unit may be omitted if the signals generated by the signal generator are strong enough. Therefore, the invention is to be accorded the broadest interpretation of the appended claims to encompass all equivalent structures and assemblies.

## Claims

1. An anal incontinence treatment apparatus, comprising
an adjustable restriction device (2) adapted to engage the colon or rectum or anus of a patient, who suffers from anal incontinence, to form a restricted faecal passageway in the colon or rectum or anus, and
an implantable adjustment device (5) for adjusting the restriction device (2) to restrict the colon or rectum to close the faecal passageway, or release the colon or rectum or anus to open the faecal passageway, when the restriction device is implanted in the patient, wherein the adjustment device comprises:
a hydraulic means for operating the adjustment device, comprising:
a first adjustable reservoir (16) containing hydraulic fluid,
a second adjustable reservoir (14) operatively connected to the first reservoir such the changes in the amount of fluid in the second reservoir causes larger changes in the amount of fluid in the first reservoir and corresponding changes in the restriction of the fecal passageway,
a third reservoir (10) adapted for subcutaneous implantation,
a hydraulic tube hydraulically interconnecting the second and third reservoirs, separating the second and third reservoirs from each other, wherein
the third reservoir is adjustable to change the amount of fluid therein to cause fluid flow in the hydraulic tube and a change of amount of fluid in the second reservoir, **characterised in that** the volume of said third reservoir is smaller than the volume of said first reservoir.

2. An apparatus according to claim 1, further comprising an operation device for transporting fluid between the third and the second reservoirs.

3. An apparatus according to claim 2, wherein the operation device comprises a pump.

4. An apparatus according to claim 2 or 3, wherein the operation device is manually operated.

5. An apparatus according to claim 2 or 3, wherein the operation device comprises a motor, preferably an electric motor.

6. An apparatus according to claim 5, wherein the motor is reversible.

7. An apparatus according to claim 5 or 6, further comprising a gearing connected between the motor and the adjustment device.

8. An apparatus according to claim 2, herein the operation device is adapted to operate the adjustment device (5) by using the hydraulic fluid of the first reservoir (16).

9. An apparatus according to claim 8, wherein the reservoir (16) contains a predetermined amount of hydraulic fluid.

10. An apparatus according to claim 9, wherein the reservoir (16) comprises first and second wall portions and is adapted to provide relative displacement between the first and second wall portions of the reservoir, in order to change the volume of the reservoir.

11. An apparatus according to claim 10, wherein said relative displacement is performed in response to the pressure in the reservoir.

12. An apparatus according to claim 11, further comprising an alarm adapted to generate an alarm signal in response to the lapse of a predetermined time period during which the pressure controlling in the third reservoir (16) exceeds a predetermined high value.

13. An apparatus according to claim 2, wherein the operation device comprises magnetic means, electric means or manual manipulation means or a combination thereof.

14. An apparatus according to claim 1, wherein the second reservoir (14) defines a chamber containing servo fluid, and comprises first and second wall portions of the second reservoir, which are displaceable relative to each other to change the volume of the chamber of the second reservoir.

15. An apparatus according to claim 14, wherein the third reservoir (10) defines a chamber for the further predetermined amount of fluid and the hydraulic operation device is adapted to change the volume of the chamber and thereby control the amount of fluid in the second reservoir (14).

16. An apparatus according to claim 15, wherein the third reservoir (10) comprises first and second wall portions, which are displaceable relative to each other to change the volume of the chamber of the third reservoir.

17. An apparatus according to claim 16, wherein the third (10) increases the amount of fluid in the second reservoir (14) in response to a predetermined first displacement of the first wall portion of the third reservoir relative to the second wall portion of the third reservoir and decreases the amount of fluid in the second reservoir in response to a predetermined second displacement of the first wall portion of the fluid third reservoir to the second wall portion of the third reservoir.

18. An apparatus according to claim 1, wherein the adjustment device (5) comprises a first reservoir (16) implantable in the patient and containing a predetermined amount of hydraulic fluid, and a conduit providing fluid connection between the first reservoir and the restriction device (2), and operates by distributing hydraulic fluid through the conduit between the first reservoir and the restriction device, the conduit and restriction adjustment device being devoid of any non-return valve to permit free flow of hydraulic fluid in both directions in the conduit.

19. An apparatus according to claim 18, wherein the first reservoir (16) forms a fluid chamber with a variable volume, and the adjustment device is adapted to distribute fluid from the chamber to the restriction device (2) by reduction of the volume of the chamber and to withdraw fluid from the restriction device by expansion of the volume of the chamber.

20. An apparatus according to claim 1, further comprising an injection port subcutaneously implantable in the patient and in fluid communication with the third resevoir (10).

21. An apparatus according to claim 20, wherein the injection port is integrated in the third reservoir (10).

22. An apparatus according to claim 1, wherein the restriction device comprises an elongated restriction member and forming means for forming the restriction member into at least a substantially closed loop around the colon or rectum, the loop defining a restriction opening, whereby the adjustment device is adapted to adjust the restriction member in the loop to change the size of the restriction opening.

23. An apparatus according to claim 22, wherein the forming means forms the restriction member (2) into a loop having a predetermined size or a size selected from several predetermined sizes.

24. An apparatus according to claim 22, wherein the adjustment device is adapted to change the size of the restriction opening such that the outer circumferential confinement surface of the restriction member is changed.

25. An apparatus according to claim 22, wherein the adjustment device is adapted to change the size of the restriction opening such that the outer circumferential confinement surface of the restriction member is unchanged.

26. An apparatus according to claim 22, wherein the restriction member is non-inflatable, and the adjustment device is adapted to adjust the restriction member in said loop.

27. An apparatus according to claim 13, wherein the adjustment device mechanically adjusts the restriction member.

28. An apparatus according to claim 26, wherein the adjustment device hydraulically adjusts the non-inflatable restriction member.

29. An apparatus according to claim 27 or 28, wherein the elongated restriction member (204) is flexible, and the adjustment device (210) is adapted to pull a first portion (204A) of the flexible restriction member from a second portion (204B) of the flexible restriction member opposite the first portion in the loop to squeeze the colon (206) or rectum between two opposite lengths of the elongated flexible restriction member to restrict the passageway, and to release the colon or rectum from the flexible restriction member to enlarge the passageway.

30. An apparatus according to claim 1, wherein the hydraulically operated adjustment device mechanically adjusts the restriction device.

31. An apparatus according to claim 18 or 30, wherein the restriction device (212) comprises at least two elements (214) to be placed on different sides of the colon (206) or rectum, and the adjustment device is adapted to squeeze the colon or rectum between the elements to restrict the faecal passageway in the colon or rectum, and to release the colon or rectum from the elements to enlarge the faecal passageway (Figs. 10A, 10B).

32. An apparatus according to claim 18 or 30 wherein the restriction device (218) comprises at least two articulated clamping elements (220) to be positioned on opposite or different sides of the colon (206) or rectum, and the adjustment device (222) is adapted to turn the clamping elements toward each other to clamp the colon or rectum between the clamping elements to restrict the faecal passageway in the colon or rectum, and to turn the clamping elements away from each other to release the colon or rectum from the elements to enlarge the faecal passageway (Fig. 11).

33. An apparatus according to claim 18 or 30, wherein the restriction device is adapted to bend a portion of the colon or rectum.

34. An apparatus according to claim 33, wherein the restriction device (224) comprises at least two bending members (226-230) to be positioned on opposite sides of the colon (206) or rectum and to be displaced relative to each other along the faecal passageway in the colon or rectum, and the adjustment device (232) is adapted to move the bending members against the colon or rectum to bend the colon or rectum to restrict the faecal passageway in the colon or rectum, and to move the bending members away from the colon or rectum to release the colon or rectum from the bending members to enlarge the faecal passageway (Figs. 12A-12C).

35. An apparatus according to claim 34, wherein the bending members comprise rollers.

36. An apparatus according to claim 18 or 30, wherein the restriction device is adapted to rotate a portion of the colon or rectum.

37. An apparatus according to any of the preceding claims, further comprising a wireless remote control (44,126,132-144) for non-invasively controlling fecal passageway when restricting the restriction device.

38. An apparatus according to claim 37, wherein the remote control comprises an external wireless hand-held remote control unit which is manually operable by the patient to control the restriction device to squeeze or release the colon or rectum.

39. An apparatus according to claim 37, wherein the remote control (44,126,132-144) comprises an external signal transmitter (132,136), receiver or transceiver and a signal receiver (134,138), transmitter or transceiver implantable in the patient.

40. An apparatus according to claim 39, wherein the signal receiver (134,138) and/or transmitter comprises a control unit (138) adapted to control the operation device (6,8;10;12;10,14;76-86) in response to a control signal received from the signal transmitter (132, 136).

41. An apparatus according to claim 40, further comprising an implantable energizer unit (136) for providing energy to energy consuming implantable components of the apparatus.

42. An apparatus according to claim 2 or 41, wherein the operation device comprises a motor and/or pump (44, 6, 12) for operating the adjustment device.

43. An apparatus according to claims 42, wherein the control unit (138) is adapted to power the motor (44) with energy provided by the energizer unit (136) in response to a control signal received from the signal transmitter (132,136).

44. An apparatus according to claim 43, wherein the remote control (44,126,132-144) comprises wireless energy transfer means for transferring energy from outside the patient's body to energy consuming implantable components of the apparatus.

45. An apparatus according to claim 41 and 44, wherein the energy transfer means comprises an implantable energizer unit (126), which is adapted to transform energy from the control signal, as it is transmitted to the signal receiver (134,138), into electric energy.

46. An apparatus according to claim 45, wherein the operation device (6;8;10;12;10,14;76-86) comprises a motor (44), and the wireless energy transfer means is adapted to directly power the motor with transferred energy.

47. An apparatus according to claim 45 or 46, wherein the energy transferred by the wireless energy transfer means comprises a signal.

48. An apparatus according to claim 47, wherein the signal comprises a wave signal.

49. An apparatus according to claim 45 or 46, wherein the energy transferred by the wireless energy transfer means comprises an electric field or a magnetic field or a combination thereof.

50. An apparatus according to claim 47, wherein the signal is analog or digital or a combination thereof.

51. An apparatus according to claim 39 or 40, wherein the signal transmitter (132,136) and signal receiver (134,138) are adapted to transmit and receive an analog or digital signal or a combination thereof.

52. An apparatus according to claim 50 or 51, wherein the signal comprises analog or digital pulses.

53. An apparatus according to any of claims 50-52, wherein the analog or digital signal comprises a magnetic field or an electric field or a combination thereof.

54. An apparatus according to claim 39 or 40, wherein the signal transmitter (132,136) and signal receiver (134,138) are adapted to transmit and receive a wave signal.

55. An apparatus according to claim 47 or 54, wherein the wave signal comprises an electromagnetic wave signal, a sound wave signal or a carrier wave signal for a remote control signal or a combination thereof.

56. An apparatus according to claim 55, wherein the carrier signal is frequency, amplitude or frequency and amplitude modulated.

57. An apparatus according to claim 47 or 50 wherein the energy transfer means transfers the energy from the signal into a direct, pulsating direct or alternating current or a combination thereof.

58. An apparatus according to claim 37, wherein the remote control (44,126,132-144) is capable of obtaining information related to important parameters of the apparatus from inside the patient's body and of commanding the adjustment device (5) to adjust the restriction device (2) in response to obtained information.

59. An apparatus according to claim 37, wherein the remote control is capable of obtaining information related to the faecal passageway in the colon or rectum and of commanding the adjustment device to adjust the restriction device in response to obtained information.

60. An apparatus according to claim 1 or 18 or 30, further comprising an implantable source of energy for powering the operation device and/or for energizing other energy consuming components of the apparatus, wherein the energy from the source of energy is releasable from outside the patient's body.

61. An apparatus according to claim 1 or 18 or 30, further comprising an energy transmission device for wireless transmission of energy.

62. An apparatus according to claim 60 and 61, wherein the energy transmission device transmits energy of a first form, and further comprising an energy transforming device implantable in the patient for transforming the energy of the first form into energy of a second form, to be supplied to the source of energy and/or other implantable energy consuming parts of the apparatus.

63. An apparatus according to claim 62, wherein the energy of the second form is different than the energy of the first form.

64. An apparatus according to claim 62, wherein the energy transmission device functions different from the energy transforming device.

65. An apparatus according to claim 61, further comprising an implantable motor or pump for operating the adjustment device, wherein the energy transmission device is adapted to transmit wireless energy in the form of an magnetic field or electromagnetic waves or field for direct power of the motor or pump, as the wireless energy is being transmitted.

66. An apparatus according to claim 40 and 61, wherein the energy transmission device transmits energy by at least one signal separate from the control signal.

67. An apparatus according to claim 66, further comprising an implantable stabiliser for stabilising the energy of the first or second form.

68. An apparatus according to claim 67, wherein the energy of the second form comprises electric current and the stabiliser comprises at least one capacitor.

69. An apparatus according to any of claims 60,62-64, wherein the source of energy comprises a battery, accumulator, capacitor or a combination thereof.

70. An apparatus according to claim 1 or 18 or 30, further comprising a control device adapted to produce wireless energy for directly powering the energy consuming components of the apparatus.

71. An apparatus according to claim 1 or 18 or 30, further comprising an implantable energy transforming device for transforming wireless energy directly or indirectly into energy different than the wireless energy for operation of the restriction device.

72. An apparatus according to claim 70 or 71, wherein the wireless energy comprises a wave signal including a sound wave signal, an ultrasound wave signal, an electromagnetic wave signal, an infrared light signal, a visible light signal, an ultra violet light signal, a laser light signal, a micro wave signal, a radio wave signal, an x-ray radiation signal or a gamma radiation signal.

73. An apparatus according to claim 1 or 18 or 30, further comprising an energy transfer means (22,326,332-344) for wireless transfer of energy from outside the patient's body to the r energy consuming implantable components of the apparatus.

74. An apparatus according to claim 70, wherein the control device is adapted to produce wireless energy in the form of a train of energy pulses.

75. An apparatus according to claim 44 or 73, wherein the energy transfer means is adapted to intermittently transfer the energy in the form of a train of energy pulses for direct use in connection with the energising of the energy consuming components of the apparatus.

76. An apparatus according to claim 70 and 62 wherein the control device is adapted to control the energy transforming device to produce the energy of the second form in a train of energy pulses for direct use in connection with the operation of the adjustment device.

77. An apparatus according to claim 75 or 76, wherein the energy transfer device is adapted to transfer electric energy, and further comprising an implantable capacitor for producing the train of energy pulses.

78. An apparatus according to claim 77 or 68,69, wherein the capacitor has a capacity less than 0,1 µF.

79. An apparatus according to claim 73, further comprising an implantable motor (22) or pump for operating the adjustment device (12;52;66;90,92;104;110), wherein the energy transfer means is adapted to directly power the motor or pump with transferred energy.

80. An apparatus according to claim 65 or 79, wherein the pump is not a plunger type of pump.

81. An apparatus according to claim 1 or 18 or 30, wherein the adjustment device is adapted to adjust the restriction device in a non-manual, non-thermal or non-magnetic manner.

82. An apparatus according to any one of the preceding claims, wherein the energy consuming parts of the apparatus are electrically powered.

83. An apparatus according to claim 1 or 65, wherein the operation of the adjustment device is unpowerable by static permanent magnetic energy.

84. An apparatus according to claim 1 or 18 or 30, wherein the adjustment device is adapted to non-invasively operate the restriction device.

85. An apparatus according to claim 1 or 18 or 30, wherein the adjustment device is operable to adjust the restriction device to steplessly change the restriction of the faecal passageway.

86. An apparatus according to claim 1 or 18, wherein hydraulic fluid having a viscosity which changes when the hydraulic fluid is exposed to energy different than thermal energy.

87. An apparatus according to claim 86, wherein the viscosity of the hydraulic fluid changes when the fluid is exposed to electric energy.

88. An apparatus according to claim 1, further comprising an adjustment device for adjusting the restriction device to change the restriction of the faecal passageway, wherein the adjustment device is adapted to mechanically adjust the restriction device, or adapted to hydraulically adjust the restriction device by using hydraulic means which is devoid of hydraulic fluid of the kind having a viscosity that substantially increases when exposed to heat or a magnetic field.

89. An apparatus according to claim 1, further comprising a control device for controlling the restriction device.

90. An apparatus according to claim 89, wherein the control device comprises an internal control unit implantable in the patient for controlling the restriction device.

91. An apparatus according to claim 90, wherein the internal control unit is programmable.

92. An apparatus according to claim 91, wherein the control device comprises an external control unit outside the patient's body, the implantable internal control unit being programmable by the external control unit.

93. An apparatus according to claim 89, wherein the control device comprises an external control unit outside the patient's body for wirelessly controlling the restriction device.

94. An apparatus according to claim 93, wherein the external control unit is programmable.

95. An apparatus according to any one of the preceding claims, further comprising at least one implantable sensor for sensing at least one physical parameter of the patient.

96. An apparatus according to claim 95, wherein the sensor is adapted to directly or indirectly sense as the physical parameter the horisontal position of the patient.

97. An apparatus according to claim 95, wherein the sensor comprises a pressure sensor for directly or indirectly sensing as the physical parameter the pressure against the restriction device or part of the human body.

98. An apparatus according to claim 97, wherein the restriction device is adapted to enlarge the faecal passageway in the colon or rectum in response to the pressure sensor sensing a predetermined pressure.

99. An apparatus according to any one of claims 95-96, further comprising a control device for controlling the restriction device in response to signals from the sensor.

100. An apparatus according to claim 99, wherein the control device comprises an internal control unit implantable in the patient and directly controlling the restriction device in response to signals from the sensor.

101. An apparatus according to claim 100, wherein the control device comprises an external control unit outside the patient's body for controlling the restriction device in response to signals from the sensor.

102. An apparatus according to claim 100, wherein the control device comprises an external control unit outside the patient's body for manually controlling the restriction device in response to information from the sensor.

103. An apparatus according to claim 89, further comprising an implantable source of energy, wherein the control device is operable from outside the patient's body for controlling the source of energy to release energy for use in connection with the operation of the prosthesis, when the prosthesis is implanted.

104. An apparatus according to claim 103, wherein the source of energy is intended to be external to the patient's body, and the control device is adapted to control the external source of energy to release wireless energy for use in connection with the operation of the restriction device.

105. An apparatus according to claim 103, wherein the control device controls the source of energy to release magnetic energy, non-magnetic energy, electromagnetic energy, non-electromagnetic energy, kinetic energy, non-kinetic energy, sonic energy, non-sonic energy, thermal energy or non-thermal energy.

106. An apparatus according to claim 1 or 18 or 30, wherein the operation of the adjustment device is powered by magnetic energy, non-magnetic energy, electromagnetic energy, non-electromagnetic energy, kinetic energy, non-kinetic energy, sonic energy, non-sonic energy, thermal energy or non-thermal energy.

107. An apparatus according to claim 103, wherein the control device controls the source of energy to release energy for a determined time period.

108. An apparatus according to claim 103 and 74, wherein the control device controls the source of energy to release energy in a determined number of energy pulses.

109. An apparatus according to claim 103, wherein the control device is adapted to control the source of energy to release energy in a non-invasive manner.

110. An apparatus according to claim 1 or 18 or 30, further comprising implantable electrical components including at least one voltage level guard.

111. An apparatus according to claim 1 or 18 or 30, further comprising implantable electrical components including a single voltage level guard.

112. An apparatus according to claim 110 or 111, wherein the electrical components are devoid of any current detector and/or charge level detector.

113. An apparatus according to any of claims 60, 77, 62, 68 or 69 and 110-112, further comprising an implantable capacitor or accumulator, wherein the charge or discharge of the capacitor or accumulator is controlled by use of the voltage level guard.

114. An apparatus according to claim 40, 45 or 66, wherein the control signal comprises a wave signal including a sound wave signal, an ultrasound wave signal, an electromagnetic wave signal, an infrared light signal, a visible light signal, an ultra violet light signal, a laser light signal, a micro wave signal, a radio wave signal, an x-ray radiation signal or a gamma radiation signal.

115. An apparatus according to any of the preceding claims, further comprising a switch implantable in the patient for directly or indirectly switching the operation of the restriction device.

116. An apparatus according to claim 121 and 66, further comprising a source of energy implantable in the patient for supplying energy for the operation of the restriction device, wherein the switch is operated by the energy supplied by the energy transmission device to switch from an off mode, in which the source of energy is not in use, to an on mode, in which the source of energy supplies energy for the operation of the restriction device.

117. An apparatus according to claim 115 and 61, further comprising a source of energy implantable in the patient for supplying energy for the operation of the restriction device, and a remote control for controlling the supply of energy of the implantable source of energy, wherein the switch is operated by the energy supplied by the energy transmission device to switch from an off mode, in which the remote control is prevented from controlling the source of energy and the source of energy is not in use, to a standby mode, in which the remote control is permitted to control the source of energy to supply energy for the operation of the restriction device.

118. An apparatus according to claim 115 and 62, further comprising a source of energy implantable in the patient for supplying energy for the operation of the restriction device, wherein the switch is operated by the energy supplied by the energy transforming device to switch from an off mode, in which the source of energy is not in use, to an on mode, in which the source of energy supplies energy for the operation of the restriction device.

119. An apparatus according to claim 116 and 62, further comprising a source of energy implantable in the patient for supplying energy for the operation of the restriction device, and a remote control for controlling the supply of energy of the implantable source of energy, wherein the switch is operated by the energy supplied by the energy transforming device to switch from an off mode, in which the remote control is prevented from controlling the source of energy and the source of energy is not in use, to a standby mode, in which the remote control is permitted to control the source of energy to supply energy for the operation of the restriction device.

120. An apparatus according to claim 1 or 18 or 30, wherein the restriction device is operable to perform a reversible function.

121. An apparatus according to claim 42 or 120, further comprising a reversing device implantable in the patient for reversing the function performed by the restriction device.

122. An apparatus according to claim 121, wherein the control device controls the reversing device to reverse the function performed by the restriction device.

123. An apparatus according to claim 121, wherein the reversing device comprises hydraulic means including a valve for shifting the flow direction of a flowing fluid in the hydraulic means.

124. An apparatus according to claim 121, wherein the reversing device comprises a mechanical reversing device.

125. An apparatus according to claim 121, wherein the reversing device comprises a switch.

126. An apparatus according to claim 125, wherein the switch of the reversing device is operable by the released energy.

127. An apparatus according to claim 126, wherein the control device controls the operation of the switch of the reversing device by shifting polarity of the released energy supplied to the switch.

128. An apparatus according to claim 121, wherein the operation device comprises a motor, and the reversing device reverses the motor.

129. An apparatus according to any of the preceding claims, wherein the restriction device is embedded in a soft or gel-like material.

130. An apparatus according to claim 129, wherein the restriction device is embedded in a silicone material having hardness less than 20 Shore.

131. An apparatus according to claim 62, wherein the energy transforming means or device is designed to be implanted subcutaneously or in the abdomen, thorax or cephalic region of the patient.

132. An apparatus according to any of the preceding claims, wherein the adjustment device is adapted to adjust the restriction device such that the restriction device provides a predetermined contraction of the faecal passageway that is satisfactory for the patient.

133. An apparatus according to claim 1, wherein the adjustment device is adapted to adjust the restriction device in a non-flux magnetic, non-thermal or non-viscosity changing manner.

## Patentansprüche

1. Vorrichtung zur Behandlung von Analinkontinenz, die umfasst:
eine einstellbare Verengungseinrichtung (2), die geeignet ist, um mit dem Kolon oder Rektum oder Anus eines Patienten in Eingriff zu kommen, der an Analinkontinenz leidet, um einen verengten Fäkaldurchlass in dem Kolon oder Rektum oder Anus auszubilden,
eine implantierbare Einstelleinrichtung (5), zur Einstellung der Verengungseinrichtung (2), um das Kolon oder Rektum oder den Anus zu verengen, um den Fäkaldurchlass zu verschließen, oder das Kolon oder Rektum oder den Anus freizugeben, um den Fäkaldurchlass zu öffnen, wenn die Verengungseinrichtung in dem Patienten implantiert ist, wobei die Einstelleinrichtung umfasst:
eine hydraulische Einrichtung für das Betätigen der Einstelleinrichtung, die umfasst:
einen ersten einstellbaren Behälter (16), der Hydraulikfluid enthält,
einen zweiten einstellbaren Behälter (14), der so wirkend mit dem ersten Behälter verbunden ist, dass die Veränderungen bei der Menge an Fluid in dem zweiten Behälter größere Veränderungen bei der Menge an Fluid in dem ersten Behälter und entsprechende Veränderungen bei der Verengung des Fäkaldurchlasses bewirken,
einen dritten Behälter (10), der für die subkutane Implantation geeignet ist,
einen Hydraulikschlauch, der den zweiten und dritten Behälter hydraulisch verbindet, den zweiten und dritten Behälter voneinander trennt, wobei der dritte Behälter einstellbar ist, um die Menge an Fluid darin zu verändern, um einen Fluidfluss in dem Hydraulikschlauch und eine Veränderungen bei der Menge an Fluid in dem zweiten Behälter zu bewirken, **dadurch gekennzeichnet, dass** das Volumen des dritten Behälters kleiner ist als das Volumen des ersten Behälters.

2. Vorrichtung nach Anspruch 1, die ferner eine Betätigungseinrichtung für das Transportieren des Fluids zwischen dem dritten und dem zweiten Behälter umfasst.

3. Vorrichtung nach Anspruch 2, wobei die Betätigungseinrichtung eine Pumpe umfasst.

4. Vorrichtung nach Anspruch 2 oder 3, wobei die Betätigungseinrichtung manuell betätigt wird.

5. Vorrichtung nach Anspruch 2 oder 3, wobei die Betätigungseinrichtung einen Motor, vorzugsweise einen Elektromotor, umfasst.

6. Vorrichtung nach Anspruch 5, wobei der Motor umkehrbar ist.

7. Vorrichtung nach Anspruch 5 oder 6, die ferner ein Getriebe umfasst, das zwischen dem Motor und der Einstelleinrichtung angeschlossen ist.

8. Vorrichtung nach Anspruch 2, wobei hierin die Betätigungseinrichtung geeignet ist, um die Einstelleinrichtung (5) zu betätigen, indem das Hydraulikfluid des ersten Behälters (16) verwendet wird.

9. Vorrichtung nach Anspruch 8, wobei der Behälter (16) eine vorgegebene Menge an Hydraulikfluid enthält.

10. Vorrichtung nach Anspruch 9, wobei der Behälter (16) erste und zweite Wandabschnitte umfasst und geeignet ist, um eine relative Verschiebung zwischen den ersten und zweiten Wandabschnitten des Behälters bereitzustellen, um das Volumen des Behälters zu verändern.

11. Vorrichtung nach Anspruch 10, wobei die relative Verschiebung in Reaktion auf den Druck in dem Behälter durchgeführt wird.

12. Vorrichtung nach Anspruch 11, die ferner eine Alarmeinrichtung umfasst, die geeignet ist, um in Reaktion auf den Ablauf einer vorgegebenen Zeitspanne, während der die Drucksteuerung in dem dritten Behälter (10) einen vorgegebenen hohen Wert übersteigt, ein Alarmsignal zu erzeugen.

13. Vorrichtung nach Anspruch 2, wobei die Betätigungseinrichtung eine magnetische Einrichtung, eine elektrische Einrichtung oder eine manuelle Bedieneinrichtung oder eine Kombination daraus umfasst.

14. Vorrichtung nach Anspruch 1, wobei der zweite Behälter (14) eine Kammer umgrenzt, die Servo-Fluid enthält, und erste und zweite Wandabschnitte des zweiten Behälters umfasst, die relativ zueinander verschoben werden können, um das Volumen der Kammer des zweiten Behälters zu ändern.

15. Vorrichtung nach Anspruch 14, wobei der dritte Behälter (10) eine Kammer für die weitere vorgegebene Menge an Fluid umgrenzt und die hydraulische Betätigungseinrichtung geeignet ist, um das Volumen der Kammer zu verändern und **dadurch** die Menge an Fluid in dem zweiten Behälter (14) zu steuern.

16. Vorrichtung nach Anspruch 15, wobei der dritte Behälter (10) erste und zweite Wandabschnitte umfasst, die relativ zueinander verschoben werden können, um das Volumen der Kammer des dritten Behälters zu ändern.

17. Vorrichtung nach Anspruch 16, wobei der dritte Behälter (10) die Menge an Fluid in dem zweiten Behälter (14) in Reaktion auf eine vorgegebene erste Verschiebung des ersten Wandabschnitts des dritten Behälters relativ zu dem zweiten Wandabschnitt des dritten Behälters vergrößert, und die Menge an Fluid in dem zweiten Behälter in Reaktion auf eine vorgegebene zweite Verschiebung des ersten Wandabschnitts des dritten Behälters zu dem zweiten Wandabschnitt des dritten Behälters verringert.

18. Vorrichtung nach Anspruch 1, wobei die Einstelleinrichtung (5) einen ersten Behälter (16) umfasst, der in den Patienten implantiert werden kann und eine vorgegebene Menge an Hydraulikfluid enthält, und eine Leitung, welche die Fluidverbindung zwischen dem ersten Behälter und der Verengungseinrichtung (2) bereitstellt, und die arbeitet, indem Hydraulikfluid durch die Leitung zwischen dem ersten Behälter und der Verengungseinrichtung verteilt wird, wobei die Leitung und die Verengungseinrichtung kein Rückschlagventil aufweisen, um den freien Fluss des Hydraulikfluids in beide Richtungen in der Leitung zu ermöglichen.

19. Vorrichtung nach Anspruch 18, wobei der erste Behälter (16) eine Fluidkammer mit einem variablen Volumen formt und die Einstelleinrichtung geeignet ist, um durch Verringerung des Volumens der Kammer Fluid von der Kammer zu der Verengungseinrichtung (2) zu verteilen, und um durch Ausdehnung des Volumens der Kammer Fluid von der Verengungseinrichtung abzuziehen.

20. Vorrichtung nach Anspruch 1, die ferner einen Injektionsanschluss umfasst, der subkutan in den Patienten implantiert werden kann und in Fluidverbindung mit dem dritten Behälter (10) steht.

21. Vorrichtung nach Anspruch 20, wobei der Injektionsanschluss in den dritten Behälter (10) integriert ist.

22. Vorrichtung nach Anspruch 1, wobei die Verengungseinrichtung ein längliches Verengungselement und eine Formeinrichtung zum Formen des Verengungselementes zu wenigstens einer im Wesentlichen geschlossenen Schleife um das Kolon oder Rektum herum umfasst, wobei die Schleife eine Verengungsöffnung bildet und die Einstelleinrichtung geeignet ist, um das Verengungselement in der Schleife einzustellen, um die Größe der Verengungsöffnung zu ändern.

23. Vorrichtung nach Anspruch 22, wobei die Formeinrichtung das Verengungselement (2) zu einer Schleife mit einer vorgegebenen Größe oder einer Größe formt, die aus mehreren vorgegebenen Größen ausgewählt wird.

24. Vorrichtung nach Anspruch 22, wobei die Einstelleinrichtung geeignet ist, um die Größe der Verengungsöffnung so zu ändern, dass die Außenumfangs-Begrenzungsfläche des Verengungselementes verändert wird.

25. Vorrichtung nach Anspruch 22, wobei die Einstelleinrichtung geeignet ist, um die Größe der Verengungsöffnung so zu ändern, dass die Außenumfangs-Begrenzungsfläche des Verengungselementes unverändert bleibt.

26. Vorrichtung nach Anspruch 22, wobei das Verengungselement nicht aufblasbar ist und die Einstelleinrichtung geeignet ist, um das Verengungselement in der Schleife einzustellen.

27. Vorrichtung nach Anspruch 13, wobei die Einstelleinrichtung das Verengungselement mechanisch einstellt.

28. Vorrichtung nach Anspruch 26, wobei die Einstelleinrichtung das nicht aufblasbare Verengungselement hydraulisch einstellt.

29. Vorrichtung nach Anspruch 27 oder 28, wobei das längliche Verengungselement (204) flexibel ist und die Einstelleinrichtung (210) geeignet ist, um einen ersten Abschnitt (204A) des flexiblen Verengungselements von einen zweiten Abschnitt (204B) des flexiblen Verengungselements, das dem ersten Abschnitt in der Schleife gegenüberliegt, abzuziehen, um das Kolon (206) oder Rektum zwischen den gegenüberliegenden Längen des länglichen flexiblen Verengungselements zusammenzudrücken, um den Durchlass zu verengen, und um das Kolon oder Rektum von dem flexiblen Verengungselement freizugeben, um den Durchlass zu vergrößern.

30. Vorrichtung nach Anspruch 1, wobei die hydraulisch betätigte Einstelleinrichtung die Verengungseinrichtung mechanisch einstellt.

31. Vorrichtung nach Anspruch 18 oder 30, wobei die Einstelleinrichtung (212) mindestens zwei Elemente (214) umfasst, die an zwei verschiedenen Seiten des Kolons (206) oder Rektums angeordnet werden sollen, und die Einstelleinrichtung ist geeignet, um das Kolon oder Rektum zwischen den Elementen zusammenzudrücken, um den Fäkaldurchlass in dem Kolon oder Rektum zu verengen, und um das Kolon oder Rektum von den Elementen freizugeben, um den Fäkaldurchlass zu vergrößern (Fig. 10A, 10B).

32. Vorrichtung nach Anspruch 18 oder 30, wobei die Einstelleinrichtung (218) mindestens zwei gelenkige Klemmelemente (220) umfasst, die an gegenüberliegenden oder verschiedenen Seiten des Kolons (206) oder Rektums positioniert werden sollen, und die Einstelleinrichtung (222) ist geeignet, um die Klemmelemente zueinander zu drehen, um das Kolon oder Rektum zwischen den Klemmelementen zu klemmen, um den Fäkaldurchlass in dem Kolon oder Rektum zu verengen, und um die Klemmelemente voneinander weg zu drehen, um das Kolon oder Rektum von den Elementen freizugeben, um den Fäkaldurchlass zu vergrößern (Fig. 11).

33. Vorrichtung nach Anspruch 18 oder 30, wobei die Verengungseinrichtung geeignet ist, um einen Abschnitt des Kolons oder Rektums zu biegen.

34. Vorrichtung nach Anspruch 33, wobei die Verengungseinrichtung (224) mindestens zwei Biegeelemente (226 - 230) umfasst, die an gegenüberliegenden Seiten des Kolons (206) oder Rektums positioniert werden sollen und die relativ zueinander entlang dem Fäkaldurchlass in dem Kolon oder Rektum verschoben werden sollen, und die Einstelleinrichtung (232) ist geeignet, um die Biegeelemente zu dem Kolon oder Rektum zu bewegen, um das Kolon oder Rektum zu biegen, um den Fäkaldurchlass in dem Kolon oder Rektum zu verengen, und um die Biegeelemente von dem Kolon oder Rektum weg zu bewegen, um das Kolon oder Rektum von den Biegeelementen freizugeben, um den Fäkaldurchlass zu vergrößern (Fig. 12A - 12C).

35. Vorrichtung nach Anspruch 34, wobei die Biegeelemente Rollen umfassen.

36. Vorrichtung nach Anspruch 18 oder 30, wobei die Verengungseinrichtung geeignet ist, um einen Abschnitt des Kolons oder Rektums zu drehen.

37. Vorrichtung nach einem der vorstehenden Ansprüche, die ferner eine drahtlose Fernsteuerung (44, 126, 132 - 144) für das nichtinvasive Steuern des Fäkaldurchlasses während des Verengens der Verengungseinrichtung umfasst.

38. Vorrichtung nach Anspruch 37, wobei die Fernsteuerung eine externe drahtlose Handheld-Fernsteuereinheit umfasst, die von dem Patienten manuell bedient werden kann, um die Verengungseinrichtung zu steuern, um das Kolon oder Rektum zusammenzudrücken oder freizugeben.

39. Vorrichtung nach Anspruch 37, wobei die Fernsteuerung (44, 126, 132 - 144) einen externen Signalsender (132, 136), -empfänger oder Sendeempfänger und einen Signalempfänger (134, 138), -sender oder Sendeempfänger umfasst, die in den Patienten implantiert werden können.

40. Vorrichtung nach Anspruch 39, wobei der Signalempfänger (134, 138) und/oder - sender eine Steuereinheit (138) umfasst, die geeignet ist, um die Betätigungseinrichtung (6, 8; 10; 12; 10, 14; 76 - 86) in Reaktion auf ein Steuersignal zu steuern, das von dem Signalsender (132, 136) empfangen wird.

41. Vorrichtung nach Anspruch 40, die ferner eine implantierbare Energieversorgungseinheit (136) für die Bereitstellung von Energie für die Energie verbrauchenden Komponenten der Vorrichtung umfasst.

42. Vorrichtung nach Anspruch 2 oder41, wobei die Betätigungseinrichtung einen Motor und/oder eine Pumpe (44, 6, 12) zum Betätigen der Einstelleinrichtung umfasst.

43. Vorrichtung nach Anspruch 42, wobei die Steuereinheit (138) geeignet ist, um den Motor (44) in Reaktion auf ein von dem Signalsender (132, 136) empfangenes Steuersignal mit Energie anzutreiben, die durch die Energieversorgungseinheit (136) bereitgestellt wird.

44. Vorrichtung nach Anspruch 43, wobei die Fernsteuerung (44, 126, 132 - 144) eine drahtlose Energieübertragungseinrichtung umfasst, um die Energie von außerhalb des Köpers des Patienten an Energie verbrauchende implantierbare Komponenten der Vorrichtung zu übertragen.

45. Vorrichtung nach Anspruch 41 und 44, wobei die Energieübertragungseinrichtung eine implantierbare Energieversorgungseinheit (126) umfasst, die geeignet ist, um Energie aus dem Steuersignal, wie es an den Signalempfänger (134, 138) gesendet wird, in elektrische Energie umzuwandeln.

46. Vorrichtung nach Anspruch 45, wobei die Betätigungseinrichtung (6; 8; 10; 12; 10, 14; 76 - 86) einen Motor umfasst und die drahtlose Energieübertragungseinrichtung geeignet ist, um den Motor direkt mit übertragener Energie anzutreiben.

47. Vorrichtung nach Anspruch 45 oder 46, wobei die Energie, die durch die drahtlose Energieübertragungseinrichtung übertragen wird, ein Signal umfasst.

48. Vorrichtung nach Anspruch 47, wobei das Signal ein Wellensignal umfasst.

49. Vorrichtung nach Anspruch 45 oder 46, wobei die Energie, die durch die drahtlose Energieübertragungseinrichtung übertragen wird, ein elektrisches Feld oder ein magnetisches Feld oder eine Kombination daraus umfasst.

50. Vorrichtung nach Anspruch 47, wobei das Signal analog oder digital oder eine Kombination daraus ist.

51. Vorrichtung nach Anspruch 39 oder 40, wobei der Signalsender (132, 136) und der Signalempfänger (134, 138) geeignet sind, um ein analoges oder digitales Signal oder eine Kombination daraus zu senden und zu empfangen.

52. Vorrichtung nach Anspruch 50 oder 51, wobei das Signal analoge oder digitale Impulse umfasst.

53. Vorrichtung nach einem der Ansprüche 50 - 52, wobei das analoge oder digitale Signal ein magnetisches Feld oder ein elektrisches Feld oder eine Kombination daraus umfasst.

54. Vorrichtung nach Anspruch 39 oder 40, wobei der Signalsender (132, 136) und der Signalempfänger (134, 138) geeignet sind, um ein Wellensignal zu senden und zu empfangen.

55. Vorrichtung nach Anspruch 47 oder 54, wobei das Wellensignal ein elektromagnetisches Wellensignal, ein Schallwellensignal oder ein Trägerwellensignal für ein Fernsteuersignal oder eine Kombination daraus umfasst.

56. Vorrichtung nach Anspruch 55, wobei das Trägersignal frequenz-, amplituden- oder frequenz- und amplitudenmoduliert ist.

57. Vorrichtung nach Anspruch 47 oder 50, wobei die Energieübertragungseinrichtung die Energie aus dem Signal in einen Gleich-, pulsierenden Gleich- oder Wechselstrom oder eine Kombination daraus überträgt.

58. Vorrichtung nach Anspruch 37, wobei die Fernsteuerung (44, 126, 132 - 144) in der Lage ist, Informationen bezüglich wichtiger Parameter der Vorrichtung aus dem Inneren des Körpers des Patienten zu beziehen und die Einstelleinrichtung (5) in Reaktion auf bezogene Informationen anzuweisen, die Verengungseinrichtung (2) einzustellen.

59. Vorrichtung nach Anspruch 37, wobei die Fernsteuerung in der Lage ist, Informationen bezüglich des Fäkaldurchlasses in dem Kolon oder Rektum zu beziehen und die Einstelleinrichtung in Reaktion auf bezogene Informationen anzuweisen, die Verengungseinrichtung einzustellen.

60. Vorrichtung nach Anspruch 1 oder 18 oder 30, die ferner eine implantierbare Energiequelle zum Antreiben der Betätigungseinrichtung und/oder zum Versorgen anderer Energie verbrauchender Komponenten der Vorrichtung mit Energie umfasst, wobei die Energie von der Energiequelle von außerhalb des Körpers des Patienten freigegeben werden kann.

61. Vorrichtung nach Anspruch 1 oder 18 oder 30, die ferner eine Energiesendeeinrichtung für das drahtlose Senden von Energie umfasst.

62. Vorrichtung nach Anspruch 60 oder 61, wobei die Energiesendeeinrichtung Energie einer ersten Form sendet, und die ferner eine Energieumwandlungseinrichtung umfasst, die in den Patienten implantiert werden kann, um die Energie der ersten Form in Energie einer zweiten Form umzuwandeln, die der Energiequelle und/oder anderen implantierbaren Energie verbrauchenden Teilen der Vorrichtung zugeführt werden soll.

63. Vorrichtung nach Anspruch 62, wobei die Energie der zweiten Form sich von der Energie der ersten Form unterscheidet.

64. Vorrichtung nach Anspruch 62, wobei die Energiesendeeinrichtung anders arbeitet als die Energieumwandlungseinrichtung.

65. Vorrichtung nach Anspruch 61, die ferner einen implantierbaren Motor oder eine Pumpe zum Betätigen der Einstelleinrichtung umfasst, wobei die Energiesendeeinrichtung geeignet ist, um drahtlose Energie in Form eines magnetischen Feldes oder elektromagnetischer Wellen oder eines elektromagnetischen Feldes zu senden, um den Motor oder die Pumpe direkt anzutreiben, während die drahtlose Energie gesendet wird.

66. Vorrichtung nach Anspruch 40 oder 61, wobei die Energiesendeeinrichtung Energie mittels wenigstens eines Signals sendet, das von dem Steuersignal getrennt ist.

67. Vorrichtung nach Anspruch 66, die ferner einen implantierbaren Stabilisator zum Stabilisieren der Energie der ersten oder zweiten Form umfasst.

68. Vorrichtung nach Anspruch 67, wobei die Energie der zweiten Form elektrischen Strom umfasst und der Stabilisator wenigstens einen Kondensator umfasst.

69. Vorrichtung nach einem der Ansprüche 60, 62 - 64, wobei die Energiequelle eine Batterie, einen Akkumulator, einen Kondensator oder eine Kombination daraus umfasst.

70. Vorrichtung nach Anspruch 1 oder 18 oder 30, die ferner eine Steuereinrichtung umfasst, die geeignet ist, um drahtlose Energie für das direkte Antreiben der Energie verbrauchenden Komponenten der Vorrichtung zu erzeugen.

71. Vorrichtung nach Anspruch 1 oder 18 oder 30, die ferner eine implantierbare Energieumwandlungseinrichtung für das direkte oder indirekte Umwandeln von drahtloser Energie in Energie umfasst, die sich von der drahtlosen Energie zur Betätigung der Verengungseinrichtung unterscheidet.

72. Vorrichtung nach Anspruch 70 oder 71, wobei die drahtlose Energie ein Wellensignal umfasst, das ein Schallwellensignal, ein Ultraschallwellensignal, ein elektromagnetisches Wellensignal, ein Infrarotlichtsignal, ein sichtbares Lichtsignal, ein Ultraviolettlichtsignal, ein Laserlichtsignal, ein Mikrowellensignal, ein Funkwellensignal, ein Röntgenstrahlungssignal oder ein Gammastrahlungssignal einschließt.

73. Vorrichtung nach Anspruch 1 oder 18 oder 30, die ferner eine Energieübertragungseinrichtung (22, 326, 332 - 344) für die drahtlose Übertragung von Energie von außerhalb des Körpers des Patienten zu den Energie verbrauchenden implantierbaren Komponenten der Vorrichtung umfasst.

74. Vorrichtung nach Anspruch 70, wobei die Steuereinrichtung geeignet ist, um drahtlose Energie in Form einer Reihe von Energieimpulsen zu erzeugen.

75. Vorrichtung nach Anspruch 44 oder 73, wobei die Energieübertragungseinrichtung geeignet ist, um die Energie intermittierend in Form einer Reihe von Energieimpulsen zur direkten Verwendung im Zusammenhang mit der Energieversorgung der Energie verbrauchenden Komponenten der Vorrichtung zu übertragen.

76. Vorrichtung nach Anspruch 70 und 62, wobei die Steuereinrichtung geeignet ist, um die Energieumwandlungseinrichtung so zu steuern, dass sie die Energie der zweiten Form in einer Reihe von Energieimpulsen zur direkten Verwendung im Zusammenhang mit der Betätigung der Einstelleinrichtung erzeugt.

77. Vorrichtung nach Anspruch 75 oder 76, wobei die Energieübertragungseinrichtung geeignet ist, um elektrische Energie zu übertragen, und die ferner einen implantierbaren Kondensator für das Erzeugen der Reihe von Energieimpulsen umfasst.

78. Vorrichtung nach Anspruch 77 oder 68, 69, wobei der Kondensator eine Kapazität von weniger als 0,1 µF hat.

79. Vorrichtung nach Anspruch 73, die ferner einen implantierbaren Motor (22) oder eine Pumpe für das Betätigen der Einstelleinrichtung (12; 52; 66; 90, 92; 104; 110) umfasst, wobei die Energieübertragungseinrichtung geeignet ist, um den Motor oder die Pumpe direkt mit übertragener Energie anzutreiben.

80. Vorrichtung nach Anspruch 65 oder 79, wobei die Pumpe keine Kolbenpumpe ist.

81. Vorrichtung nach den Ansprüchen 1 oder 18 oder 30, wobei die Einstelleinrichtung geeignet ist, um die Verengungseinrichtung nicht-manuell, nicht-thermisch oder nicht-magnetisch einzustellen.

82. Vorrichtung nach einem der vorstehenden Ansprüche, wobei die Energie verbrauchenden Bauteile der Vorrichtung elektrisch angetrieben werden.

83. Vorrichtung nach einem der Anspruch 1 oder 65, wobei der Betrieb der Einstellungseinrichtung nicht durch statische Permanentmagnetenergie angetrieben werden kann.

84. Vorrichtung nach Anspruch 1 oder 18 oder 30, wobei die Einstellungseinrichtung geeignet ist, um die Verengungseinrichtung nichtinvasiv zu betätigen.

85. Vorrichtung nach Anspruch 1 oder 18 oder 30, wobei die Einstelleinrichtung so betrieben werden kann, dass sie die Verengungseinrichtung so einstellt, dass die Verengung des Fäkaldurchlasses stufenlos geändert wird.

86. Vorrichtung nach Anspruch 1 oder 18, wobei die Viskosität des Hydraulikfluids sich ändert, wenn das Hydraulikfluid anderer Energie als thermischer Energie ausgesetzt wird.

87. Vorrichtung nach einem der Anspruch 86, wobei das Hydraulikfluid eine Viskosität aufweist die sich ändert, wenn das Fluid elektrischer Energie ausgesetzt wird.

88. Vorrichtung nach Anspruch 1, die ferner eine Einstelleinrichtung für das Einstellen der Verengungseinrichtung umfasst, um die Verengung des Fäkaldurchlasses zu verändern, wobei die Einstelleinrichtung geeignet ist, um die Verengungseinrichtung mechanisch einzustellen, oder geeignet ist, um die Verengungseinrichtung hydraulisch einzustellen, indem eine hydraulische Einrichtung verwendet wird, die über kein Hydraulikfluid von der Art verfügt, das eine Viskosität aufweist, die sich im Wesentlichen erhöht, sobald es Wärme oder einem magnetischen Feld ausgesetzt wird.

89. Vorrichtung nach Anspruch 1, die ferner eine Steuereinrichtung zum Steuern der Verengungseinrichtung umfasst.

90. Vorrichtung nach Anspruch 89, wobei die Steuereinrichtung eine interne Steuereinheit umfasst, die in den Patienten implantiert werden kann, um die Verengungseinrichtung zu steuern.

91. Vorrichtung nach Anspruch 90, wobei die interne Steuereinheit programmierbar ist.

92. Vorrichtung nach Anspruch 91, wobei die Steuereinrichtung eine externe Steuereinheit außerhalb des Körpers des Patienten umfasst und die implantierbare interne Steuereinheit durch die externe Steuereinheit programmiert werden kann.

93. Vorrichtung nach Anspruch 89, wobei die Steuereinrichtung eine externe Steuereinheit außerhalb des Körpers des Patienten für das drahtlose Steuern der Verengungseinrichtung umfasst.

94. Vorrichtung nach Anspruch 93, wobei die externe Steuereinheit programmierbar ist.

95. Vorrichtung nach einem der vorstehenden Ansprüche, die ferner wenigstens einen implantierbaren Sensor umfasst, der wenigstens einen physikalischen Parameter des Patienten wahrnimmt.

96. Vorrichtung nach Anspruch 95, wobei der Sensor geeignet ist, um die horizontale Position des Patienten direkt oder indirekt als den physikalischen Parameter wahrzunehmen.

97. Vorrichtung nach Anspruch 95, wobei der Sensor einen Drucksensor umfasst, der den Druck auf die Verengungseinrichtung oder einen Teil des menschlichen Körpers direkt oder indirekt als den physikalischen Parameter wahrnimmt.

98. Vorrichtung nach Anspruch 97, wobei die Verengungseinrichtung geeignet ist, um den Fäkaldurchlass in dem Kolon oder Rektum in Reaktion darauf zu vergrößern, dass der Drucksensor einen vorgegebenen Druck wahrnimmt.

99. Vorrichtung nach einem der Ansprüche 95 - 96, die ferner eine Steuereinrichtung für das Steuern der Verengungseinrichtung in Reaktion auf Signale von dem Sensor umfasst.

100. Vorrichtung nach Anspruch 99, wobei die Steuereinrichtung eine interne Steuereinheit umfasst, die in den Patienten implantiert werden kann und die Verengungseinrichtung in Reaktion auf Signale von dem Sensor direkt steuert.

101. Vorrichtung nach Anspruch 100, wobei die Steuereinrichtung eine externe Steuereinheit außerhalb des Körpers des Patienten umfasst, um die Verengungseinrichtung in Reaktion auf Signale von dem Sensor zu steuern.

102. Vorrichtung nach Anspruch 100, wobei die Steuereinrichtung eine externe Steuereinheit außerhalb des Körpers des Patienten zum manuellen Steuern der Verengungseinrichtung in Reaktion auf Informationen von dem Sensor umfasst.

103. Vorrichtung nach Anspruch 89, die ferner eine implantierbare Energiequelle umfasst, wobei die Steuereinrichtung von außerhalb des Körpers des Patienten betätigt werden kann, um die Energiequelle so zu steuern, dass sie Energie zur Verwendung im Zusammenhang mit der Betätigung der Prothese freigibt, wenn die Prothese implantiert ist.

104. Vorrichtung nach Anspruch 103, wobei die Energiequelle sich außerhalb des Körpers des Patienten befinden soll und die Steuereinrichtung geeignet ist, um die externe Energiequelle so zu steuern, dass sie drahtlose Energie zur Verwendung im Zusammenhang mit der Betätigung der Verengungseinrichtung freigibt.

105. Vorrichtung nach Anspruch 103, wobei die Steuereinrichtung die Energiequelle steuert, um magnetische Energie, nicht-magnetische Energie, elektromagnetische Energie, nicht-elektromagnetische Energie, kinetische Energie, nichtkinetische Energie, Schallenergie, Nicht-Schallenergie, thermische Energie oder nicht-thermische Energie freizugeben.

106. Vorrichtung nach Anspruch 1 oder 18 oder 30, wobei die Betätigung der Einstelleinrichtung mit magnetischer Energie, nicht-magnetischer Energie, elektromagnetischer Energie, nicht-elektromagnetischer Energie, kinetischer Energie, nichtkinetischer Energie, Schallenergie, Nicht-Schallenergie, thermischer Energie oder nicht-thermischer Energie angetrieben wird.

107. Vorrichtung nach Anspruch 103, wobei die Steuereinrichtung die Energiequelle so steuert, dass sie Energie über einen vorgegebenen Zeitraum freigibt.

108. Vorrichtung nach Anspruch 103 und 74, wobei die Steuereinrichtung die Energiequelle so steuert, dass sie Energie in einer vorgegebenen Anzahl von Energieimpulsen freigibt.

109. Vorrichtung nach Anspruch 103, wobei die Steuereinrichtung geeignet ist, um die Energiequelle so zu steuern, dass sie Energie auf nichtinvasive Weise freizugeben.

110. Vorrichtung nach Anspruch 1 oder 18 oder 30, die ferner implantierbare elektrische Komponenten einschließlich wenigstens eines Spannungspegelbegrenzers umfasst.

111. Vorrichtung nach Anspruch 1 oder 18 oder 30, die ferner implantierbare elektrische Komponenten einschließlich eines einzelnen Spannungspegelbegrenzers umfasst.

112. Vorrichtung nach Anspruch 110 oder 111, wobei die elektrischen Komponenten keinen Stromdetektor und/oder Ladungspegeldetektor aufweisen.

113. Vorrichtung nach Anspruch 60, 77, 62, 68 oder 69 und 110 - 112, die ferner einen implantierbaren Kondensator oder Akkumulator umfasst, wobei die Ladung oder Entladung des Kondensators oder Akkumulators durch den Einsatz des Spannungspegelbegrenzers gesteuert wird.

114. Vorrichtung nach Anspruch 40, 45 oder 66, wobei das Steuersignal ein Wellensignal umfasst, das ein Schallwellensignal, ein Ultraschallwellensignal, ein elektromagnetisches Wellensignal, ein Infrarotlichtsignal, ein sichtbares Lichtsignal, ein Ultraviolettlichtsignal, ein Laserlichtsignal, ein Mikrowellensignal, ein Funkwellensignal, ein Röntgenstrahlungssignal oder ein Gammastrahlungssignal einschließt.

115. Vorrichtung nach einem der vorstehenden Ansprüche, die ferner einen Schalter umfasst, der in den Patienten implantiert werden kann, um die Betätigung der Verengungseinrichtung direkt oder indirekt zu schalten.

116. Vorrichtung nach Anspruch 121 und 66, die ferner eine Energiequelle umfasst, die in den Patienten implantiert werden kann, um Energie für die Betätigung der Verengungseinrichtung zuzuführen, wobei der Schalter mit der durch die Energiesendeeinrichtung zugeführten Energie betätigt wird, um von einem Aus-Modus, in dem die Energiequelle nicht in Betrieb ist, in einen Ein-Modus umzuschalten, in dem die Energiequelle Energie für die Betätigung der Verengungseinrichtung zuführt.

117. Vorrichtung nach Anspruch 115 oder 61, die ferner eine Energiequelle, die in den Patienten implantiert werden kann, um Energie für die Betätigung der Verengungseinrichtung zuzuführen, sowie eine Fernsteuerung für das Steuern der Zufuhr von Energie der implantierbaren Energiequelle umfasst, wobei der Schalter mit der durch die Energiesendeeinrichtung zugeführten Energie betätigt wird, um von einem Aus-Modus, in dem verhindert wird, dass die Fernsteuerung die Energiequelle steuert, und in dem die Energiequelle nicht in Betrieb ist, in einen Bereitschaftsmodus umzuschalten, in dem die Fernsteuerung die Energiequelle steuern kann, um Energie für die Betätigung der Verengungseinrichtung zuzuführen.

118. Vorrichtung nach Anspruch 115 und 62, die ferner eine Energiequelle umfasst, die in den Patienten implantiert werden kann, um Energie für die Betätigung der Verengungseinrichtung zuzuführen, wobei der Schalter mit der durch die Energieumwandlungseinrichtung zugeführten Energie betätigt wird, um von einem Aus-Modus, in dem die Energiequelle nicht in Betrieb ist, in einen Ein-Modus umzuschalten, in dem die Energiequelle Energie für die Betätigung der Verengungseinrichtung zuführt.

119. Vorrichtung nach Anspruch 116 und 62, die ferner eine Energiequelle, die in den Patienten implantiert werden kann, um Energie für die Betätigung der Verengungseinrichtung zuzuführen, sowie eine Fernsteuerung für das Steuern der Zufuhr von Energie der implantierbaren Energiequelle umfasst, wobei der Schalter mit der durch die Energieumwandlungseinrichtung zugeführten Energie betätigt wird, um von einem Aus-Modus, in dem die Fernsteuerung daran gehindert wird, die Energiequelle zu steuern, und in dem die Energiequelle nicht in Betrieb ist, in einen Bereitschaftsmodus umzuschalten, in dem die Fernsteuerung die Energiequelle steuern kann, um Energie für die Betätigung der Verengungseinrichtung zuzuführen.

120. Vorrichtung nach Anspruch 1 oder 18 oder 30, wobei die Verengungseinrichtung so betrieben werden kann, dass sie eine umkehrbare Funktion ausführt.

121. Vorrichtung nach Anspruch 42 oder 120, die ferner eine Umkehreinrichtung umfasst, die in den Patienten implantiert werden kann, um die durch die Verengungseinrichtung ausgeführte Funktion umzukehren.

122. Vorrichtung nach Anspruch 121, wobei die Steuereinrichtung die Umkehreinrichtung so steuert, dass sie die durch die Verengungseinrichtung ausgeführte Funktion umkehrt.

123. Vorrichtung nach Anspruch 121, wobei die Umkehreinrichtung eine hydraulische Einrichtung umfasst, die ein Ventil zum Wechseln der Strömungsrichtung eines strömenden Fluids in der hydraulischen Einrichtung enthält.

124. Vorrichtung nach Anspruch 121, wobei die Umkehreinrichtung eine mechanische Umkehreinrichtung umfasst.

125. Vorrichtung nach Anspruch 121, wobei die Umkehreinrichtung einen Schalter umfasst.

126. Vorrichtung nach Anspruch 125, wobei der Schalter der Umkehreinrichtung durch die freigegebene Energie betätigt werden kann.

127. Vorrichtung nach Anspruch 126, wobei die Steuereinrichtung die Betätigung des Schalters der Umkehreinrichtung steuert, indem sie die Polarität der dem Schalter zugeführten freigegebenen Energie wechselt.

128. Vorrichtung nach Anspruch 121, wobei die Betätigungseinrichtung einen Motor umfasst und die Umkehreinrichtung den Motor umschaltet.

129. Vorrichtung nach einem der vorstehenden Ansprüche, wobei die Verengungseinrichtung in ein weiches oder gelartiges Material eingebettet ist.

130. Vorrichtung nach Anspruch 129, wobei die Verengungseinrichtung in ein Silikonmaterial eingebettet ist, das eine Härte von weniger als 20 Shore aufweist.

131. Vorrichtung nach Anspruch 62, wobei die Energieumwandlungseinrichtung so ausgeführt ist, dass sie subkutan oder in den Bauch-, Thorax- oder Schädelbereich des Patienten implantiert wird.

132. Vorrichtung nach einem der vorstehenden Ansprüche, wobei die Einstelleinrichtung geeignet ist, um die Verengungseinrichtung so einzustellen, dass die Verengungseinrichtung eine vorgegebene Kontraktion des Fäkaldurchlasses bereitstellt, die für den Patienten zufriedenstellend ist.

133. Vorrichtung nach Anspruch 1, wobei die Einstelleinrichtung geeignet ist, um die Verengungseinrichtung ohne Magnetfluss-, thermische oder Viskositätsänderung einzustellen.

## Revendications

1. Appareil de traitement de l'incontinence anale, comprenant :
un dispositif réglable de rétrécissement (2) destiné à coopérer avec le côlon ou le rectum ou l'anus d'un patient qui souffre d'incontinence anale, pour former un passage fécal rétréci dans le côlon, le rectum ou l'anus, et
un dispositif implantable (5) d'ajustement du dispositif de rétrécissement (2) afin que le côlon ou le rectum soit rétréci pour fermer le passage fécal ou pour libérer le côlon ou le rectum ou l'anus pour ouvrir le passage fécal lorsque le dispositif de rétrécissement est implanté dans le patient, dans lequel le dispositif d'ajustement comprend :
un dispositif hydraulique de manoeuvre du dispositif d'ajustement, comprenant :
un premier réservoir réglable (16) contenant du fluide hydraulique,
un second réservoir réglable (14) raccordé pendant le fonctionnement au premier réservoir afin que les changements de quantité de fluide dans le second réservoir provoquent des changements plus importants de la quantité de fluide dans le premier réservoir et des changements correspondants du rétrécissement du passage fécal,
un troisième réservoir (10) destiné à une implantation sous-cutanée, et
un tube hydraulique raccordant hydrauliquement les second et troisième réservoirs, et séparant l'un de l'autre les second et troisième réservoirs, et tel que
le troisième réservoir est réglable afin que la quantité de fluide qu'il contient soit modifiée pour provoquer une circulation de fluide dans le tube hydraulique et un changement de la quantité de fluide dans le second réservoir,
**caractérisé en ce que** le volume du troisième réservoir est inférieur au volume du premier réservoir.

2. Appareil selon la revendication 1, comprenant en outre un dispositif de manoeuvre destiné à transporter du fluide entre le troisième et le second réservoir.

3. Appareil selon la revendication 2, dans lequel le dispositif de manoeuvre comporte une pompe.

4. Appareil selon la revendication 2 ou 3, dans lequel le dispositif de manoeuvre est manoeuvré à la main.

5. Appareil selon la revendication 2 ou 3, dans lequel le dispositif de manoeuvre comporte un moteur, de préférence un moteur électrique.

6. Appareil selon la revendication 5, dans lequel le moteur est réversible.

7. Appareil selon la revendication 5 ou 6, comprenant en outre une transmission à engrenage raccordée entre le moteur et le dispositif d'ajustement.

8. Appareil selon la revendication 2, dans lequel le dispositif de manoeuvre est destiné à manoeuvrer le dispositif d'ajustement (5) par utilisation du fluide hydraulique du premier réservoir (16).

9. Appareil selon la revendication 8, dans lequel le réservoir (16) contient une quantité prédéterminée de fluide hydraulique.

10. Appareil selon la revendication 9, dans lequel le réservoir (16) comprend des première et seconde parties de paroi et est destiné à assurer un déplacement relatif des première et seconde parties de paroi du réservoir afin de modifier le volume du réservoir.

11. Appareil selon la revendication 10, dans lequel le déplacement relatif est réalisé en fonction de la pression dans le réservoir.

12. Appareil selon la revendication 11, comprenant en outre une alarme destinée à créer un signal d'alarme à la suite de l'écoulement d'une période prédéterminée pendant laquelle le réglage de pression dans le troisième réservoir (16) dépasse une valeur élevée prédéterminée.

13. Appareil selon la revendication 2, dans lequel le dispositif de manoeuvre comprend un dispositif magnétique, un dispositif électrique ou un dispositif de manipulation manuel ou une de leurs combinaisons.

14. Appareil selon la revendication 1, dans lequel le second réservoir (14) délimite une chambre qui contient du fluide d'asservissement, et comporte des première et seconde parties de paroi du second réservoir, qui peuvent être déplacées l'une par rapport à l'autre pour changer le volume de la chambre du second réservoir.

15. Appareil selon la revendication 14, dans lequel le troisième réservoir (10) délimite une chambre pour la quantité prédéterminée supplémentaire de fluide et le dispositif hydraulique de manoeuvre est destiné à changer le volume de la chambre et ainsi à régler la quantité de fluide dans le second réservoir (14).

16. Appareil selon la revendication 15, dans lequel le troisième réservoir (10) comprend des première et seconde parties de paroi qui sont mobiles l'une par rapport à l'autre afin que le volume de la chambre du troisième réservoir soit modifié

17. Appareil selon la revendication 16, dans lequel le troisième réservoir (10) augmente la quantité de fluide dans le second réservoir (14) à la suite d'un premier déplacement prédéterminé de la première partie de paroi du troisième réservoir par rapport à la seconde partie de paroi du troisième réservoir et réduit la quantité de fluide dans le second réservoir à la suite d'un second déplacement prédéterminé de la première partie de paroi du troisième réservoir vers la seconde partie de paroi du troisième réservoir.

18. Appareil selon la revendication 1, dans lequel le dispositif d'ajustement (5) comprend un premier réservoir (16) implantable dans le patient et contenant une quantité prédéterminée de fluide hydraulique, et un conduit assurant un raccordement pour le fluide entre le premier réservoir et le dispositif de rétrécissement (2), et il fonctionne par distribution de fluide hydraulique par l'intermédiaire du conduit entre le premier réservoir et le dispositif de rétrécissement, le conduit et le dispositif d'ajustement de rétrécissement n'ayant aucun clapet de retenue pour permettre un écoulement libre du fluide hydraulique dans les deux sens dans le conduit.

19. Appareil selon la revendication 18, dans lequel le premier réservoir (16) forme une chambre de fluide de volume variable, et le dispositif d'ajustement est destiné à distribuer du fluide de la chambre au dispositif de rétrécissement (2) par réduction du volume de la chambre et à extraire du fluide du dispositif de rétrécissement par dilatation du volume de la chambre.

20. Appareil selon la revendication 1, comprenant en outre un canal d'injection implantable de manière sous-cutanée dans le patient et communiquant pour la circulation du fluide avec le troisième réservoir (10).

21. Appareil selon la revendication 20, dans lequel le canal d'injection est intégré au troisième réservoir (10).

22. Appareil selon la revendication 1, dans lequel le dispositif de rétrécissement comprend un organe allongé de rétrécissement et un dispositif de formation destiné à former l'organe de rétrécissement à la configuration d'une boucle pratiquement fermée autour du côlon ou du rectum, la boucle délimitant une ouverture de rétrécissement, de sorte que le dispositif d'ajustement est destiné à ajuster l'organe de rétrécissement dans la boucle pour changer la dimension de l'ouverture de rétrécissement.

23. Appareil selon la revendication 22, dans lequel le dispositif de formation forme avec l'organe de rétrécissement (2) une boucle de dimension prédéterminée ou de dimension sélectionnée parmi quelques dimensions prédéterminées.

24. Appareil selon la revendication 22, dans lequel le dispositif d'ajustement est destiné à modifier la dimension de l'ouverture de rétrécissement de manière que la surface circonférentielle externe de confinement de l'organe de rétrécissement soit modifiée.

25. Appareil selon la revendication 22, dans lequel le dispositif d'ajustement est destiné à modifier la dimension de l'ouverture de rétrécissement de manière que la surface circonférentielle externe de confinement de l'organe de rétrécissement soit inchangée.

26. Appareil selon la revendication 22, dans lequel l'organe de rétrécissement n'est pas gonflable, et le dispositif d'ajustement est destiné à ajuster l'organe de rétrécissement dans la boucle.

27. Appareil selon la revendication 13, dans lequel le dispositif d'ajustement ajuste mécaniquement l'organe de rétrécissement.

28. Appareil selon la revendication 26, dans lequel le dispositif d'ajustement ajuste hydrauliquement l'organe de rétrécissement non gonflable.

29. Appareil selon la revendication 27 ou 28, dans lequel l'organe allongé de rétrécissement (204) est flexible et le dispositif d'ajustement (210) est destiné à tirer une première partie (204A) de l'organe flexible de rétrécissement depuis une seconde partie (204B) de l'organe flexible de rétrécissement opposé à la première partie dans la boucle afin de serrer le côlon (206) ou le rectum entre deux tronçons opposés de l'organe flexible allongé de rétrécissement afin de rétrécir le passage, et à libérer le côlon ou le rectum de l'organe flexible de rétrécissement pour élargir le passage.

30. Appareil selon la revendication 1, dans lequel le dispositif d'ajustement manoeuvré hydrauliquement ajuste mécaniquement le dispositif de rétrécissement.

31. Appareil selon la revendication 18 ou 30, dans lequel le dispositif de rétrécissement (212) comprend au moins deux éléments (214) destinés à être placés sur des côtés différents du côlon (206) ou du rectum, et le dispositif d'ajustement est destiné à serrer le côlon ou le rectum entre les éléments pour limiter le passage fécal dans le côlon ou le rectum et à libérer le côlon ou le rectum des éléments afin que le passage fécal s'élargisse (figures 10A, 10B).

32. Appareil selon la revendication 18 ou 30, dans lequel le dispositif de rétrécissement (218) comporte au moins deux éléments articulés de serrage (220) destinés à être placés sur des côtés différents ou opposés du côlon (206) ou du rectum, et le dispositif d'ajustement (222) est destiné à faire tourner les éléments de serrage l'un vers l'autre pour serrer le côlon ou le rectum entre les éléments de serrage et rétrécir le passage fécal dans le côlon ou le rectum, et à faire tourner les éléments de serrage à distance l'un de l'autre pour libérer le côlon ou le rectum des éléments afin que le passage fécal soit élargi (figure 11).

33. Appareil selon la revendication 18 ou 30, dans lequel le dispositif de rétrécissement est destiné à courber une partie du côlon ou du rectum.

34. Appareil selon la revendication 33, dans lequel le dispositif de rétrécissement (224) comporte au moins deux organes de courbure (226-230) destinés à être placés sur les côtés opposés du côlon (206) ou du rectum et à être déplacés l'un par rapport à l'autre le long du passage fécal dans le côlon ou le rectum, et le dispositif d'ajustement (232) est destiné à déplacer les organes de courbure contre le côlon ou le rectum pour courber le côlon ou le rectum et limiter le passage fécal dans le côlon ou le rectum, et à écarter les organes de courbure du côlon ou du rectum pour libérer le côlon ou le rectum des organes de courbure pour élargir le passage fécal (figures 12A-12C).

35. Appareil selon la revendication 34, dans lequel les organes de courbure comportent des rouleaux.

36. Appareil selon la revendication 18 ou 30, dans lequel le dispositif de rétrécissement est destiné à faire tourner une partie du côlon ou du rectum.

37. Appareil selon l'une quelconque des revendications précédentes, comprenant en outre une commande à distance sans fil (44, 126, 132-144) destinée à régler le passage fécal lors du rétrécissement du dispositif de rétrécissement d'une manière non traumatisante.

38. Appareil selon la revendication 37, dans lequel la commande à distance comprend une unité externe de commande à distance sans fil tenue à la main qui peut être manoeuvrée à la main par le patient pour régler le dispositif de rétrécissement afin qu'il serre le côlon ou le rectum ou le libère.

39. Appareil selon la revendication 37, dans lequel la commande à distance (44, 126, 132-144) comporte un émetteur (132, 136), récepteur ou émetteur-récepteur externe de signaux et un récepteur (134, 138), émetteur ou émetteur-récepteur de signaux implantable dans le patient.

40. Appareil selon la revendication 39, dans lequel le récepteur (134, 138) et/ou l'émetteur de signaux comporte une unité de commande (138) destinée à commander le dispositif de manoeuvre (6, 8 ; 10 ; 12 ; 10, 14 ; 76-86) en réponse à un signal de commande reçu de l'émetteur de signaux (132, 136).

41. Appareil selon la revendication 40, comprenant en outre une unité implantable à organe d'alimentation (136) destinée à transmettre de l'énergie aux éléments implantables consommateurs d'énergie de l'appareil.

42. Appareil selon la revendication 2 ou 41, dans lequel le dispositif de manoeuvre comprend un moteur et/ou une pompe (44, 6, 12) destinée à la manoeuvre du dispositif d'ajustement.

43. Appareil selon la revendication 42, dans lequel l'unité de commande (138) est destinée à alimenter le moteur (44) avec de l'énergie donnée par l'unité à organe d'alimentation (136) en fonction d'un signal de commande reçu de l'émetteur de signaux (132, 136).

44. Appareil selon la revendication 43, dans lequel la commande à distance (44, 126, 132-144) comporte un dispositif de transfert d'énergie sans fil destiné à transférer de l'énergie depuis l'extérieur du corps du patient aux composants implantables consommateurs d'énergie de l'appareil.

45. Appareil selon la revendication 41 ou 44, dans lequel le dispositif de transfert d'énergie comprend une unité implantable à organe d'alimentation (126) qui est destinée à transformer de l'énergie provenant du signal de commande tel qu'il est transmis au récepteur de signaux (134, 138) en énergie électrique.

46. Appareil selon la revendication 45, dans lequel le dispositif de manoeuvre (6 ; 8 ; 10 ; 12 ; 10, 14 ; 76-86) comporte un moteur (44), et le dispositif de transfert d'énergie sans fil est destiné à alimenter directement le moteur par l'énergie transférée.

47. Appareil selon la revendication 45 ou 46, dans lequel l'énergie transférée par le dispositif de transfert d'énergie sans fil forme un signal.

48. Appareil selon la revendication 47, dans lequel le signal est un signal ondulatoire.

49. Appareil selon la revendication 45 ou 46, dans lequel l'énergie transférée par le dispositif de transfert d'énergie sans fil comporte un champ électrique ou un champ magnétique ou une de leurs combinaisons.

50. Appareil selon la revendication 47, dans lequel le signal est analogique ou numérique ou une de leurs combinaisons.

51. Appareil selon la revendication 39 ou 40, dans lequel l'émetteur de signaux (132, 136) et le récepteur de signaux (134, 138) sont destinés à émettre et recevoir un signal analogique ou numérique ou une de leurs combinaisons.

52. Appareil selon la revendication 50 ou 51, dans lequel le signal comprend des impulsions analogiques ou numériques.

53. Appareil selon l'une quelconque des revendications 50 à 52, dans lequel le signal analogique ou numérique est un champ magnétique ou un champ électrique ou une de leurs combinaisons.

54. Appareil selon la revendication 39 ou 40, dans lequel l'émetteur de signaux (132, 136) et le récepteur de signaux (134, 138) sont destinés à émettre et recevoir un signal ondulatoire.

55. Appareil selon la revendication 47 ou 54, dans lequel le signal ondulatoire est un signal d'ondes électromagnétiques, un signal d'ondes acoustiques ou un signal d'onde porteuse destiné à un signal de commande à distance ou une de leurs combinaisons.

56. Appareil selon la revendication 55, dans lequel le signal d'onde porteuse est modulé en fréquence, en amplitude, ou en fréquence et en amplitude.

57. Appareil selon la revendication 47 ou 50, dans lequel le dispositif de transfert d'énergie transfère l'énergie du signal en un courant continu, continu-pulsé ou alternatif ou en une de leurs combinaisons.

58. Appareil selon la revendication 37, dans lequel la commande à distance (44, 126, 132-144) est destinée à obtenir des informations relatives à des paramètres importants de l'appareil depuis l'intérieur du corps du patient et à commander le dispositif d'ajustement (5) pour l'ajustement du dispositif de rétrécissement (2) en fonction des informations obtenues.

59. Appareil selon la revendication 37, dans lequel la commande à distance est capable d'obtenir des informations relatives au passage fécal formé dans le côlon ou le rectum et de commander le dispositif d'ajustement pour ajuster le dispositif de rétrécissement selon les informations obtenues.

60. Appareil selon la revendication 1 ou 18 ou 30, comprenant en outre une source implantable d'énergie destinée à alimenter le dispositif de manoeuvre et/ou à alimenter d'autres composants consommateurs d'énergie de l'appareil, et telle que l'énergie provenant de la source d'énergie peut être libérée depuis l'extérieur du corps du patient.

61. Appareil selon la revendication 1 ou 18 ou 30, comprenant en outre un dispositif de transmission d'énergie destiné à la transmission d'énergie sans fil.

62. Appareil selon la revendication 60 ou 61, dans lequel le dispositif de transmission d'énergie transmet de l'énergie d'une première forme, et comprenant en outre un dispositif de transformation d'énergie implantable dans le patient et destiné à transformer l'énergie de la première forme en énergie d'une seconde forme destinée à être transmise à la source d'énergie et/ou à d'autres parties consommatrices d'énergie et implantables de l'appareil.

63. Appareil selon la revendication 62, dans lequel l'énergie de la seconde forme est différente de l'énergie de la première forme.

64. Appareil selon la revendication 62, dans lequel le dispositif de transmission d'énergie fonctionne différemment du dispositif de transformation d'énergie.

65. Appareil selon la revendication 61, comprenant en outre une pompe ou un moteur implantable destiné à manoeuvrer le dispositif d'ajustement, et tel que le dispositif de transmission d'énergie est destiné à transmettre de l'énergie sans fil sous forme d'un champ magnétique ou d'ondes électromagnétiques ou d'un champ d'alimentation directe du moteur ou de la pompe, lorsque de l'énergie sans fil est transmise.

66. Appareil selon la revendication 40 et 61, dans lequel le dispositif de transmission d'énergie transmet de l'énergie à l'aide d'au moins un signal séparé du signal de commande.

67. Appareil selon la revendication 66, comprenant en outre un stabilisateur implantable destiné à stabiliser l'énergie de la première ou de la seconde forme.

68. Appareil selon la revendication 67, dans lequel l'énergie de la seconde forme est un courant électrique et le stabilisateur comprend au moins un condensateur.

69. Appareil selon l'une quelconque des revendications 60 et 62 à 64, dans lequel la source d'énergie comprend une batterie d'accumulateurs, un accumulateur, un condensateur ou une de leurs combinaisons.

70. Appareil selon la revendication 1 ou 18 ou 30, comprenant en outre un dispositif de commande destiné à produire de l'énergie sans fil pour l'alimentation directe des composants consommateurs d'énergie de l'appareil.

71. Appareil selon la revendication 1 ou 18 ou 30, comprenant en outre un dispositif implantable de transformation d'énergie destiné à transformer l'énergie sans fil directement ou indirectement en énergie différente de l'énergie sans fil pour la manoeuvre du dispositif de rétrécissement.

72. Appareil selon la revendication 70 ou 71, dans lequel l'énergie sans fil est un signal ondulatoire comprenant un signal d'ondes acoustiques, un signal d'ondes ultrasonores, un signal d'ondes électromagnétiques, un signal de lumière infrarouge, un signal de lumière visible, un signal de lumière ultraviolette, un signal de lumière laser, un signal d'hyperfréquences, un signal à hautes fréquences, un signal de rayonnement infrarouge ou un signal de rayonnement gamma.

73. Appareil selon la revendication 1 ou 18 ou 30, comprenant en outre un dispositif de transfert d'énergie (22, 326, 332-344) destiné au transfert sans fil d'énergie depuis l'extérieur du corps du patient vers les composants implantables consommateurs d'énergie de l'appareil.

74. Appareil selon la revendication 70, dans lequel le dispositif de commande est destiné à produire de l'énergie sans fil sous forme d'un train d'impulsions d'énergie.

75. Appareil selon la revendication 44 ou 73, dans lequel le dispositif de transfert d'énergie est destiné à transférer par intermittence l'énergie sous forme d'un train d'impulsions en énergie destiné à être utilisé directement lors de l'alimentation des composants consommateurs d'énergie de l'appareil.

76. Appareil selon les revendications 70 et 62, dans lequel le dispositif de commande est destiné à commander le dispositif de transformation d'énergie pour la production d'énergie de la seconde forme avec un train d'impulsions d'énergie destiné à être utilisé directement lors de la manoeuvre du dispositif d'ajustement.

77. Appareil selon la revendication 75 ou 76, dans lequel le dispositif de transfert d'énergie est destiné à transférer de l'énergie électrique, et comprenant en outre un condensateur implantable destiné à produire le train d'impulsions d'énergie.

78. Appareil selon la revendication 77 ou 68, 69, dans lequel le condensateur a une capacité inférieure à 0,1 µF.

79. Appareil selon la revendication 73, comprenant en outre un moteur (22) ou une pompe implantable destiné à manoeuvrer le dispositif d'ajustement (12 ; 52 ; 66 ; 90, 92 ; 104 ; 110), dans lequel le dispositif de transfert d'énergie est destiné à alimenter directement le moteur ou la pompe en énergie transférée.

80. Appareil selon la revendication 65 ou 79, dans lequel la pompe n'est pas une pompe du type à plongeur.

81. Appareil selon la revendication 1 ou 18 ou 30, dans lequel le dispositif d'ajustement est destiné à ajuster le dispositif de rétrécissement d'une manière non manuelle, non thermique ou non magnétique.

82. Appareil selon l'une quelconque des revendications précédentes, dans lequel les parties consommatrices d'énergie de l'appareil sont alimentées électriquement.

83. Appareil selon la revendication 1 ou 65, dans lequel la manoeuvre du dispositif d'ajustement ne peut pas être assurée par de l'énergie magnétique permanente statique.

84. Appareil selon la revendication 1 ou 18 ou 30, dans lequel le dispositif d'ajustement est destiné à manoeuvrer le dispositif de rétrécissement de manière non traumatisante.

85. Appareil selon la revendication 1 ou 18 ou 30, dans lequel le dispositif d'ajustement peut être manoeuvré pour l'ajustement du dispositif de rétrécissement afin qu'il fasse varier de façon continue le rétrécissement du passage fécal.

86. Appareil selon la revendication 1 ou 18, dans lequel du fluide hydraulique ayant une viscosité qui change lorsque le fluide hydraulique est exposé à de l'énergie différente de l'énergie thermique est utilisé.

87. Appareil selon la revendication 86, dans laquelle la viscosité du fluide hydraulique change lorsque le fluide est exposé à de l'énergie électrique.

88. Appareil selon la revendication 1, comprenant en outre un dispositif d'ajustement destiné à ajuster le dispositif de rétrécissement pour modifier le rétrécissement du passage fécal, dans lequel le dispositif d'ajustement est destiné à ajuster mécaniquement le dispositif de rétrécissement ou à ajuster hydrauliquement le dispositif de rétrécissement pour l'utilisation d'un dispositif hydraulique qui est dépourvu d'un fluide hydraulique du type ayant une viscosité qui augmente notablement lors de l'exposition à la chaleur ou à un champ magnétique.

89. Appareil selon la revendication 1, comprenant en outre un dispositif de commande destiné à commander le dispositif de rétrécissement.

90. Appareil selon la revendication 89, dans lequel le dispositif de commande comprend une unité interne de commande et implantable dans le patient pour la commande du dispositif de rétrécissement.

91. Appareil selon la revendication 90, dans lequel l'unité interne de commande est programmable.

92. Appareil selon la revendication 91, dans lequel le dispositif de commande comprend une unité externe de commande placée à l'extérieur du corps du patient, l'unité interne implantable de commande étant programmable par l'unité externe de commande.

93. Appareil selon la revendication 89, dans lequel le dispositif de commande comprend une unité externe de commande placée à l'extérieur du corps du patient pour la commande sans fil du dispositif de rétrécissement.

94. Appareil selon la revendication 93, dans lequel l'unité externe de commande est programmable.

95. Appareil selon l'une quelconque des revendications précédentes, comprenant en outre au moins un capteur implantable destiné à détecter au moins un paramètre physique du patient.

96. Appareil selon la revendication 95, dans lequel le capteur est destiné à détecter, directement ou indirectement, comme paramètre physique, la position horizontale du patient.

97. Appareil selon la revendication 95, dans lequel le capteur comporte un capteur de pression destiné à détecter directement ou indirectement, comme paramètre physique, la pression appliquée sur le dispositif de rétrécissement ou une partie du corps humain.

98. Appareil selon la revendication 97, dans lequel le dispositif de rétrécissement est destiné à élargir le passage fécal dans le côlon ou le rectum lorsque le capteur de pression détecte une pression prédéterminée.

99. Appareil selon l'une quelconque des revendications 95 et 96, comprenant en outre un dispositif de commande destiné à commander le dispositif de rétrécissement en fonction de signaux provenant du capteur.

100. Appareil selon la revendication 99, dans lequel le dispositif de commande comporte une unité interne de commande implantable dans le patient et commandant directement le dispositif de rétrécissement en fonction de signaux provenant du capteur.

101. Appareil selon la revendication 100, dans lequel le dispositif de commande comprend une unité externe de commande placée à l'extérieur du corps du patient et destinée à commander le dispositif de rétrécissement en fonction de signaux provenant du capteur.

102. Appareil selon la revendication 100, dans lequel le dispositif de commande comprend une unité externe de commande placée à l'extérieur du corps de patient et destinée à commander manuellement le dispositif de rétrécissement en fonction d'informations provenant du capteur.

103. Appareil selon la revendication 89, comprenant en outre une source implantable d'énergie, dans lequel le dispositif de commande est manoeuvré depuis l'extérieur du corps du patient pour la commande de la source d'énergie afin que de l'énergie soit libérée pour être utilisée lors du fonctionnement de la prothèse lorsque la prothèse est implantée.

104. Appareil selon la revendication 103, dans lequel la source d'énergie est destinée à être extérieure au corps du patient, et le dispositif de commande est destiné à commander la source extérieure d'énergie pour libérer de l'énergie sans fil destinée à être utilisée lors de la manoeuvre du dispositif de rétrécissement.

105. Appareil selon la revendication 103, dans lequel le dispositif de commande assure la commande de la source d'énergie pour libérer de l'énergie magnétique, de l'énergie non magnétique, de l'énergie électromagnétique, de l'énergie non électromagnétique, de l'énergie cinétique, de l'énergie non cinétique, de l'énergie acoustique, de l'énergie non acoustique, de l'énergie thermique ou de l'énergie non thermique.

106. Appareil selon la revendication 1 ou 18 ou 30, dans lequel la manoeuvre du dispositif d'ajustement est alimentée par de l'énergie magnétique, de l'énergie non magnétique, de l'énergie électromagnétique, de l'énergie non électromagnétique, de l'énergie cinétique, de l'énergie non cinétique, de l'énergie acoustique, de l'énergie non acoustique, de l'énergie thermique ou de l'énergie non thermique.

107. Appareil selon la revendication 103, dans lequel le dispositif de commande assure la commande de la source d'énergie pour libérer de l'énergie pendant une période déterminée.

108. Appareil selon les revendications 103 et 74, dans lequel le dispositif de commande commande la source d'énergie pour libérer de l'énergie sous forme d'un nombre déterminé d'impulsions d'énergie.

109. Appareil selon la revendication 103, dans lequel le dispositif de commande est destiné à commander la source d'énergie pour libérer de l'énergie de manière non traumatisante.

110. Appareil selon la revendication 1 ou 18 ou 30, comprenant en outre des composants électriques implantables comprenant au moins un organe de protection contre le niveau de tension.

111. Appareil selon la revendication 1 ou 18 ou 30, comprenant en outre des composants électriques implantables comprenant un seul organe de protection contre un niveau de tension.

112. Appareil selon la revendication 110 ou 111, dans lequel les composants électriques sont dépourvus de tout détecteur de courant et/ou de tout détecteur de niveau de charge.

113. Appareil selon l'une quelconque des revendications 60, 77, 62, 68 ou 69 et 110 à 112, comprenant en outre un condensateur ou accumulateur implantable, dans lequel la charge ou la décharge du condensateur ou de l'accumulateur est commandée à l'aide de l'organe de protection contre un niveau de tension.

114. Appareil selon la revendication 40, 45 ou 66, dans lequel le signal de commande comporte un signal ondulatoire comprenant un signal d'ondes acoustiques, un signal d'ondes ultrasonores, un signal d'ondes électromagnétiques, un signal de lumière infrarouge, un signal de lumière visible, un signal de lumière ultraviolette, un signal de lumière laser, un signal d'hyperfréquences, un signal à hautes fréquences, un signal de rayons X ou un signal de rayonnement gamma.

115. Appareil selon l'une quelconque des revendications précédentes, comprenant en outre un commutateur implantable dans le patient pour commuter directement ou indirectement la manoeuvre du dispositif de rétrécissement.

116. Appareil selon les revendications 121 et 66, comprenant en outre une source d'énergie implantable dans le patient pour la transmission d'énergie destinée à la manoeuvre du dispositif de rétrécissement, dans lequel le commutateur est manoeuvré par l'énergie transmise par le dispositif de transmission d'énergie pour commuter d'un état d'arrêt, dans lequel la source d'énergie n'est pas utilisée, à un état de fonctionnement, dans lequel la source d'énergie transmet de l'énergie pour la manoeuvre du dispositif de rétrécissement.

117. Appareil selon les revendications 115 et 61, comprenant en outre une source d'énergie implantable dans le patient pour transmettre de l'énergie de manoeuvre du dispositif de rétrécissement, une commande à distance destinée à commander la transmission d'énergie de la source implantable d'énergie, dans lequel le commutateur est manoeuvré par de l'énergie transmise par le dispositif de transmission d'énergie afin qu'elle commute d'un mode d'arrêt dans lequel la commande à distance ne peut pas commander la source d'énergie et la source d'énergie n'est pas utilisée, à un mode d'attente dans lequel la commande à distance peut commander la source d'énergie afin qu'elle transmette de l'énergie pour la manoeuvre du dispositif de rétrécissement.

118. Appareil selon les revendications 115 et 62, comprenant en outre une source d'énergie implantable dans le patient pour la transmission d'énergie de manoeuvre du dispositif de rétrécissement, et dans lequel le commutateur est manoeuvré par l'énergie transmise par le dispositif de transformation d'énergie afin qu'il commute d'un mode d'arrêt dans lequel la source d'énergie n'est pas utilisée à un mode de fonctionnement dans lequel la source d'énergie transmet de l'énergie pour la manoeuvre du dispositif de rétrécissement.

119. Appareil selon les revendications 116 et 62, comprenant en outre une source d'énergie implantable dans le patient pour la transmission d'énergie de manoeuvre du dispositif de rétrécissement, et une commande à distance destinée à commander la transmission d'énergie de la source d'énergie implantable, dans lequel le commutateur est manoeuvré par l'énergie transmise par le dispositif de transmission d'énergie afin qu'il commute un mode d'arrêt dans lequel la commande à distance ne peut pas commander la source d'énergie et la source d'énergie n'est pas utilisée, à un mode d'attente dans lequel la commande à distance peut commander la source d'énergie pour transmettre de l'énergie de manoeuvre du dispositif de rétrécissement.

120. Appareil selon la revendication 1 ou 18 ou 30, dans lequel le dispositif de rétrécissement peut être manoeuvré pour assurer un fonctionnement réversible.

121. Appareil selon la revendication 42 ou 120, comprenant en outre un dispositif d'inversion implantable dans le patient et destiné à inverser le fonctionnement exécuté par les dispositifs de rétrécissement.

122. Appareil selon la revendication 121, dans lequel le dispositif de commande commande le dispositif d'inversion pour inverser le fonctionnement du dispositif de rétrécissement.

123. Appareil selon la revendication 121, dans lequel le dispositif d'inversion comporte un dispositif hydraulique comprenant une soupape destinée à modifier le sens de circulation d'un fluide qui circule dans le dispositif hydraulique.

124. Appareil selon la revendication 121, dans lequel le dispositif d'inversion comprend un dispositif mécanique d'inversion.

125. Appareil selon la revendication 121, dans lequel le dispositif d'inversion comporte un commutateur.

126. Appareil selon la revendication 125, dans lequel le commutateur du dispositif d'inversion est destiné à être manoeuvré par l'énergie libérée.

127. Appareil selon la revendication 126, dans lequel le dispositif de commande assure la commande du fonctionnement du commutateur du dispositif d'inversion par changement de la polarité de l'énergie libérée transmise au commutateur.

128. Appareil selon la revendication 121, dans lequel le dispositif de manoeuvre est un moteur, et le dispositif d'inversion inverse le fonctionnement du moteur.

129. Appareil selon l'une quelconque des revendications précédentes, dans lequel le dispositif de rétrécissement est enrobé dans un matériau souple ou analogue à un gel.

130. Appareil selon la revendication 129, dans lequel le dispositif de rétrécissement est enrobé dans un matériau de silicone dont la dureté est inférieure à une dureté Shore 20.

131. Appareil selon la revendication 62, dans lequel l'organe ou dispositif de transformation d'énergie est destiné à être implanté de manière sous-cutanée ou dans l'abdomen, le thorax ou la région céphalique du patient.

132. Appareil selon l'une quelconque des revendications précédentes, dans lequel le dispositif d'ajustement est destiné à ajuster le dispositif de rétrécissement afin que le dispositif de rétrécissement assure une contraction prédéterminée du passage fécal qui est satisfaisante pour le patient.

133. Appareil selon la revendication 1, dans lequel le dispositif d'ajustement est destiné à ajuster le dispositif de rétrécissement d'une manière sans flux magnétique, non thermique ou sans changement de viscosité.
